(19) Europäisches Patentamt
European Patent Office
Office européen des brevets

(11) Veröffentlichungsnummer: **0 354 429**
**A1**

(12) **EUROPÄISCHE PATENTANMELDUNG**

(21) Anmeldenummer: **89113966.9**

(51) Int. Cl.4: **C07D 405/06 , A01N 43/653 , A01N 43/50**

(22) Anmeldetag: **28.07.89**

(30) Priorität: **10.08.88 DE 3827135**

(43) Veröffentlichungstag der Anmeldung: **14.02.90 Patentblatt 90/07**

(84) Benannte Vertragsstaaten: **BE CH DE ES FR GB IT LI NL SE**

(71) Anmelder: **BAYER AG**
**D-5090 Leverkusen 1 Bayerwerk(DE)**

(72) Erfinder: **Elbe, Hans-Ludwig, Dr.**
**Dasnöckel 59**
**D-5600 Wuppertal 11(DE)**
Erfinder: **Kranz, Eckart, Dr.**
**Am Acker 9**
**D-5600 Wuppertal 1(DE)**
Erfinder: **Brandes, Wilhelm, Dr.**
**Eichendorffstrasse 3**
**D-5653 Leichlingen 1(DE)**
Erfinder: **Dutzmann, Stefan, Dr.**
**Leinenweberweg 33**
**D-4000 Düsseldorf 13(DE)**
Erfinder: **Hänssler, Gerd, Dr.**
**Am Arenzberg 58a**
**D-5090 Leverkusen 3(DE)**

(54) **Substituierte Dioxolane, Verfahren zu ihrer Herstellung und ihre Verwendung als Schädlingsbekämpfungsmittel.**

(57) Neue substituierte Dioxolane der Formel

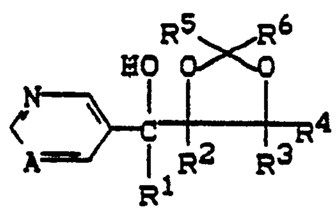

(I),

in welcher
A und $R^1$ bis $R^6$ die in der Beschreibung gegebenen Bedeutungen haben,
und deren Verwendung zur Schädlingsbekämpfung. Die neuen substituierten Dioxolane der Formel (I) können hergestellt werden, indem man geeignete Dioxolanylketone mit geeigneten Heterocyclen umsetzt und gegebenenfalls anschließend eine geeignete Säure oder ein geeignetes Metallsalz addiert.

EP 0 354 429 A1

**Substituierte Dioxolane, Verfahren zu ihrer Herstellung und ihre Verwendung als Schädlingsbekämpfungsmittel**

Die Erfindung betrifft neue substituierte Dioxolane, ein Verfahren zu ihrer Herstellung und ihre Verwendung in Schädlingsbekämpfungsmitteln.

Es ist bekannt, daß bestimmte heterocyclisch substituierte Carbinole, wie beispielsweise die Verbindung 2-(4-Chlorphenoxy)-2-methyl-1-(3-pyridyl)-1-propanol oder die Verbindung 4-(4-Chlorphenyl)-3,3-dimethyl-1-(1,2,4-triazol-1-yl)-2-butanol oder die Verbindung (4-Chlorphenyl)-(5-pyrimidyl)-methanol fungizide Eigenschaften besitzen (vgl. z.B. EP 221 844; EP 55 833; US-PS 4 417 050).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue substituierte Dioxolane der allgemeinen Formel (I),

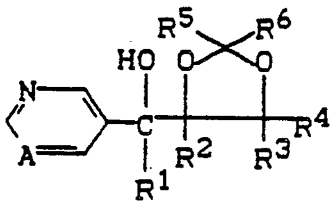

(I)

in welcher

$R^1$ für jeweils unsubstituiertes oder substituiertes Alkyl, Alkenyl, Alkinyl, Cycloalkyl oder Aryl steht,

$R^2$, $R^3$, $R^4$, $R^5$ und $R^6$ unabhängig voneinander jeweils für Wasserstoff, Alkyl oder Cycloalkyl stehen, wobei auch entweder $R^3$ und $R^4$ gemeinsam oder $R^5$ und $R^6$ gemeinsam für einen zweifach verknüpften Alkandiylrest stehen können und

A für Stickstoff oder eine CH-Gruppe steht,

sowie deren Säureadditionssalze und Metallsalzkomplexe gefunden.

Die neuen substituierten Dioxolane der Formel (I) besitzen in Abhängigkeit von der Art der Substituenten $R^1$ bis $R^6$ zwei oder mehr asymmetrisch substituierte Kohlenstoffatome und können daher in Form von optischen Isomeren oder Diastereomeren auftreten. Die Erfindung betrifft sowohl die reinen Isomeren als auch deren Gemische in unterschiedlicher Zusammensetzung.

Weiterhin wurde gefunden, daß man die neuen substituierten Dioxolane der allgemeinen Formel (I),

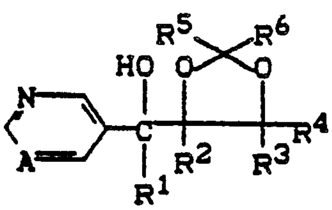

(I)

in welcher

$R^1$ für jeweils unsubstituiertes oder substituiertes Alkyl, Alkenyl, Alkinyl, Cycloalkyl oder Aryl steht,

$R^2$, $R^3$, $R^4$, $R^5$ und $R^6$ unabhängig voneinander jeweils für Wasserstoff, Alkyl oder Cycloalkyl stehen, wobei auch entweder $R^3$ und $R^4$ gemeinsam oder $R^5$ und $R^6$ gemeinsam für einen zweifach verknüpften Alkandiylrest stehen können und

A für Stickstoff oder eine CH-Gruppe steht,

sowie deren Säureadditionssalze und Metallsalzkomplexe erhält, wenn man Dioxolanylketone der Formel (II),

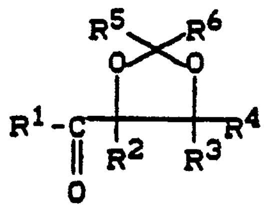

(II)

in welcher
$R^1$, $R^2$, $R^3$, $R^4$, $R^5$ und $R^6$ die oben angegebene Bedeutung haben,
mit metallorganischen Heterocyclen der Formel (III),

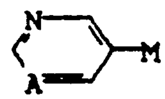

(III)

in welcher
M für ein Alkalimetallkation oder für einen Rest -Mg-Hal steht, wobei
Hal für Halogen steht und
A die oben angegebene Bedeutung hat,
gegebenenfalls in Gegenwart eines Verdünnungsmittels und gegebenenfalls in Gegenwart eines Reaktionshilfsmittels umsetzt und gegebenenfalls anschließend eine Säure oder ein Metallsalz addiert.

Schließlich wurde gefunden, daß die neuen substituierten Dioxolane der allgemeinen Formel (I) sowie deren Säureadditionssalze und Metallsalzkomplexe eine ausgezeichnete Wirksamkeit gegen Schädlinge besitzen.

Überraschenderweise zeigen die erfindungsgemäßen substituierten Dioxolane der allgemeinen Formel (I) z.B. eine erheblich bessere Wirksamkeit gegen pflanzenschädigende Pilze als die folgenden aus dem Stand der Technik bekannten, chemisch und/oder wirkungsmäßig naheliegenden Verbindungen 2-(4-Chlorphenoxy)-2-methyl-1-(3-pyridyl)-1-propanol oder die Verbindung 4-(4-Chlorphenyl)-3,3-dimethyl-1-(1,2,4-triazol-1-yl)-2-butanol oder die Verbindung (4-Chlorphenyl)-(5-pyrimidyl)-methanol.

Die erfindungsgemäßen substituierten Dioxolane sind durch die Formel (I) allgemein definiert.

Alkyl ist im folgenden, falls nicht anders definiert, z.B. geradkettiges oder verzweigtes Alkyl mit 1 bis 12, vorzugsweise 1 bis 8, insbesondere 1 bis 7 und vor allem 3 bis 7 Kohlenstoffatomen.

Alkyl kann substituiert sein durch Alkoxy, Alkylthio, Alkylsulfinyl, Alkylsulfonyl, Alkoximino, Hydroximinocyano, Halogen, Halogenalkoxy, Halogenalkylthio, Halogensulfinyl oder Halogensulfonyl mit jeweils 1 bis 8 Kohlenstoffatomen in den Alkylteilen und jeweils 1 bis 9 Halogenatomen, ferner durch Dioxolanyl, Dithiolanyl, Dioxanyl, Dithianyl, Cycloalkyl, Aryl, Aryloxy, Arylthio, Arylsulfinyl, Arylsulfonyl, Aralkyloxy, Aralkylthio, Aralkylsulfinyl oder Aralkylsulfonyl.

Alkenyl und Alkinyl sind im folgenden, falls nicht anders definiert, z.B. geradkettiges oder verzweigtes Alkenyl bzw. Alkinyl mit 2 bis 12, vorzugsweise 2 bis 8, insbesondere 2 bis 7 und vor allem 3 bis 7 Kohlenstoffatomen.

Alkenyl und Alkinyl können jeweils durch Halogen substituiert sein.

Cycloalkyl allein oder in Zusammensetzungen wie Cycloalkylalkyl enthält im allgemeinen 3 bis 7, vorzugsweise 3 bis 6 Kohlenstoffatome im Cycloalkylteil.

Aryl als solches oder in Zusammensetzungen, wie Aralkyloxyalkyl u.a. enthält im allgemeinen 6 bis 10 Kohlenstoffatome im Arylteil, wobei Phenyl-, α-Naphthyl- oder ß-Naphthylreste beispielhaft genannt seien.

Diese Reste können z.B. durch ein oder mehrere, gleiche oder verschiedene Halogenatome wie Fluor, Chlor, Brom und Jod, vorzugsweise Fluor, Chlor und Brom, Cyano, Nitro, geradkettiges oder verzweigtes Alkyl, Alkoxy, Alkylthio, Alkylsulfinyl oder Alkylsulfonyl mit 1 bis 8, vorzugsweise 1 bis 6 und insbesondere 1 bis 4 Kohlenstoffatomen im Alkylteil, geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, unsubstituiertes oder einfach oder mehrfach, gleich oder verschieden durch Halogen substituiertes, zweifach verknüpftes Dioxyalkylen, Cycloalkyl mit 3 bis 7 Kohlenstoffatomen und unsubstituiertes oder einfach oder mehrfach, gleich oder verschieden durch Halogen und/oder geradkettiges oder verzweigtes Alkyl mit 1 bis 4 Kohlenstoffatomen substituiertes Phenyl oder Phenoxy substituiert sein.

Bevorzugt sind Verbindungen der Formel (I), in denen

$R^1$ für jeweils geradkettiges oder verzweigtes Alkyl mit 1 bis 12 Kohlenstoffatomen, Alkenyl mit 2 bis 12 Kohlenstoffatomen oder Alkinyl mit 2 bis 12 Kohlenstoffatomen, für jeweils geradkettiges oder verzweigtes Alkoxyalkyl, Alkylthioalkyl, Alkylsulfinylalkyl, Alkylsulfonylalkyl, Alkoximinoalkyl, Hydroximinoalkyl oder Cyanalkyl mit jeweils 1 bis 8 Kohlenstoffatomen in den einzelnen Alkylteilen, für jeweils geradkettiges oder verzweigtes Halogenalkyl, Halogenalkoxyalkyl, Halogenalkylthioalkyl, Halogenalkylsulfinylalkyl oder Halogenalkylsulfonylalkyl mit jeweils 1 bis 8 Kohlenstoffatomen in den einzelnen Alkylteilen und jeweils 1 bis 9 gleichen oder verschiedenen Halogenatomen, für jeweils geradkettiges oder verzweigtes Halogenalkenyl oder Halogenalkinyl mit jeweils 2 bis 12 Kohlenstoffatomen und jeweils 1 bis 9 gleichen oder verschiedenen Halogenatomen, für Dioxolanylalkyl, Dithiolanylalkyl, Dioxanylalkyl oder Dithianylalkyl mit jeweils 1 bis 6 Kohlenstoffatomen im geradkettigen oder verzweigten Alkylteil oder für jeweils unsubstituiertes oder im Cycloalkylteil einfach oder mehrfach, gleich oder verschieden substituiertes Cycloalkylalkyl oder Cycloalkyl mit jeweils 3 bis 7 Kohlenstoffatomen im Cycloalkylteil und 1 bis 8 Kohlenstoffatomen im geradkettigen oder verzweigten Alkylteil steht, wobei als Substituenten im Cycloalkylteil jeweils infrage kommen: Halogen oder geradkettiges oder verzweigtes Alkyl mit 1 bis 4 Kohlenstoffatomen; oder für unsubstituiertes oder im Arylteil einfach bis mehrfach, gleich oder verschieden substituiertes Arylalkyl, Aryloxyalkyl, Arylthioalkyl, Arylsulfinylalkyl, Arylsulfonylalkyl, Aralkyloxyalkyl, Aralkylthioalkyl, Aralkylsulfinylalkyl, Aralkylsulfonylalkyl oder Aryl mit jeweils 6 bis 10 Kohlenstoffatomen im Arylteil und 1 bis 8 Kohlenstoffatomen in den einzelnen geradkettigen oder verzweigten Alkylteilen steht, wobei als Arylsubstituenten jeweils infrage kommen: Halogen, Cyano, Nitro, jeweils geradkettiges oder verzweigtes Alkyl, Alkoxy, Alkylthio, Alkylsufinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den ein einzelnen Alkylteilen, unsubstituiertes oder einfach bis mehrfach, gleich oder verschieden durch Halogen substituiertes, zweifach verknüpftes Dioxyalkylen mit 1 bis 3 Kohlenstoffatomen, Cycloalkyl mit 3 bis 7 Kohlenstoffatomen und unsubstituiertes oder einfach oder mehrfach, gleich oder verschieden durch Halogen und/oder geradkettiges oder verzweigtes Alkyl mit 1 bis 4 Kohlenstoffatomen substituiertes Phenyl oder Phenoxy;

$R^2$, $R^3$, $R^4$, $R^5$ und $R^6$ unabhängig voneinander jeweils für Wasserstoff, für geradkettiges oder verzweigtes Alkyl mit 1 bis 6 Kohlenstoffatomen oder für Cycloalkyl mit 3 bis 7 Kohlenstoffatomen stehen, wobei auch entweder $R^3$ und $R^4$ gemeinsam oder $R^5$ und $R^6$ gemeinsam für einen jeweils zweifach verknüpften Alkandiylrest mit 2 bis 6 Kohlenstoffatomen stehen können und

A für Stickstoff oder eine CH-Gruppe steht.

Besonders bevorzugt sind Verbindungen der Formel (I), in denen

$R^1$ für jeweils geradkettiges oder verzweigtes Alkyl mit 1 bis 8 Kohlenstoffatomen, Alkenyl mit 2 bis 8 Kohlenstoffatomen oder Alkinyl mit 2 bis 8 Kohlenstoffatomen, für jeweils geradkettiges oder verzweigtes Alkoxyalkyl, Alkylthioalkyl, Alkylsulfinylalkyl, Alkylsulfonylalkyl, Alkoximinoalkyl, Hydroximinoalkyl oder Cyanalkyl mit jeweils 1 bis 6 Kohlenstoffatomen in den einzelnen Alkylteilen, für jeweils geradkettiges oder verzweigtes Halogenalkyl, Halogenalkoxyalkyl, Halogenalkylthioalkyl, Halogenalkylsulfinylalkyl oder Halogenalkylsulfonylalkyl mit jeweils 1 bis 4 Kohlenstoffatomen in den einzelnen Alkylteilen und jeweils 1 bis 5 gleichen oder verschiedenen Halogenatomen, für jeweils geradkettiges oder verzweigtes Halogenalkenyl oder Halogenalkinyl mit jeweils 3 bis 8 Kohlenstoffatomen und jeweils 1 bis 5 gleichen oder verschiedenen Halogenatomen, für Dioxolanylalkyl, Dithiolanylalkyl, Dioxanylalkyl oder Dithianylalkyl mit jeweils 1 bis 4 Kohlenstoffatomen im geradkettigen oder verzweigten Alkylteil;

oder für jeweils unsubstituiertes oder im Cycloalkylteil ein- bis fünffach, gleich oder verschieden substituiertes Cycloalkylalkyl oder Cycloalkyl mit jeweils 3 bis 6 Kohlenstoffatomen im Cycloalkylteil und 1 bis 6 Kohlenstoffatomen im geradkettigen oder verzweigten Alkylteil steht, wobei als Substituenten im Cycloalkylteil jeweils infrage kommen: Fluor, Chlor oder Brom oder geradkettiges oder verzweigtes Alkyl mit 1 bis 4 Kohlenstoffatomen;

oder für jeweils unsubstituiertes oder im Arylteil ein- bis fünffach, gleich oder verschieden substituiertes Arylalkyl, Aryloxyalkyl, Arylthioalkyl, Arylsulfinylalkyl, Arylsulfonylalkyl, Aralkyloxyalkyl, Aralkylthioalkyl, Aralkylsulfinylalkyl, Aralkylsulfonylalkyl oder Aryl mit jeweils 1 bis 6 Kohlenstoffatomen in den einzelnen geradkettigen oder verzweigten Alkylteilen steht, wobei Aryl jeweils für Phenyl, α-Naphthyl oder β-Naphthyl steht, wobei als Phenylsubstituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Propan-1,3-diyl, Butan-1,4-diyl, Pentan-1,5-diyl, Dimethylpropan-1,3-diyl, Tetramethylpropan-1,3-diyl, Dimethylbutan-1,4-diyl, Tetramethylbutan-1,4-diyl, Cyclopropyl, Cyclobutyl, Cyclopentyl, Cyclohexyl, Trifluormethyl, Dichlorfluormethyl, Difluorchlormethyl, Difluor brommethyl, Trichlormethyl, Fluormethyl, Difluormethyl, Trifluorethyl, Tetrafluorethyl, Trifluorchlorethyl, Pentafluorethyl, Trifluormethoxy, Difluormethoxy, Fluormethoxy, Dichlorfluormethoxy, Difluorchlormethoxy, Difluorbrommethoxy, Trichlormethoxy, Trifluorethoxy, Tetrafluorethoxy, Pentafluorethoxy, Trifluorchlorethoxy, Trifluordichlorethoxy, Difluortrichlorethoxy, Pentachlorethoxy, Trifluormethylthio, Difluormethylthio, Fluormethylthio, Difluorchlormethylthio, Dichlorfluormethylthio, Difluorethylthio, Difluorbrommethylthio, Trichlormethylthio, Trifluorethylthio, Tetrafluorethylthio, Pentafluorethylthio, Trifluorchlorethylthio, Trifluordichlorethylthio, Pentachlorethylthio, Methylsulfinyl, Trifluormethylsulfinyl, Dichlorfluormethylsulfinyl, Difluorchlormethylsulfinyl, Fluormethylsulfinyl, Difluormethylsulfinyl, Methylsulfonyl, Trifluormethylsulfonyl, Dichlorfluormethylsulfonyl, Difluorchlormethylsulfonyl, Fluormethylsulfonyl, Difluormethylsulfonyl, Methoxycarbonyl, Ethoxycarbonyl, Methoximinomethyl, Ethoximinomethyl, Methoximinoethyl, Ethoximinoethyl, Difluordioxymethylen, Tetrafluordioxyethylen, Trifluordioxyethylen, Difluordioxyethylen, Dioxymethylen, Dioxyethylen, jeweils unsubstituiertes oder ein-bis dreifach, gleich oder verschieden durch Methyl, Fluor oder Chlor substituiertes Phenyl oder Phenoxy;

und wobei als Naphthylsubstituenten jeweils infrage kommen: Fluor, Chlor, Brom, Cyano, Methyl, Ethyl, n-oder i-Propyl;

$R^2$, $R^3$, $R^4$, $R^5$ und $R^6$ unabhängig voneinander jeweils für Wasserstoff, Methyl, Ethyl, n- oder i-Propyl, n-, i-, s- oder t-Butyl, für Cyclopentyl oder für Cyclohexyl stehen, wobei auch entweder $R^3$ und $R^4$ gemeinsam oder $R^5$ und $R^6$ gemeinsam für einen jeweils zweifach verknüpften Butan-1,4-diylrest oder Pentan-1,5-diylrest stehen können und

A für Stickstoff oder eine CH-Gruppe steht.

Ganz besonders bevorzugt sind Verbindungen der Formel (I), in denen

$R^1$ für jeweils geradkettiges oder verzweigtes Alkyl mit 1 bis 7 Kohlenstoffatomen, Alkenyl mit 2 bis 7 Kohlenstoffatomen, Alkinyl mit 2 bis 7 Kohlenstoffatomen, für jeweils geradkettiges oder verzweigtes Methoxyalkyl, Dimethoxyalkyl, Trimethoxyalkyl, Methylthioalkyl, Dimethylthioalkyl Trimethylthioalkyl, Methylsulfinylalkyl, Methylsulfonylalkyl, Methoximinoalkyl, Hydroximinoalkyl oder Cyanalkyl mit jeweils 3 bis 6 Kohlenstoffatomen im Alkylteil, für jeweils verzweigtes Halogenalkyl, Halogenmethoxyalkyl, Halogenethoxyalkyl, Halogenmethylthioalkyl, Halogenmethylsulfinylalkyl oder Halogenmethylsulfonylalkyl mit jeweils 1 bis 3 gleichen oder verschiedenen Halogenatomen, insbesondere Fluor, Chlor oder Brom und jeweils 3 bis 7 Kohlenstoffatomen im Alkylteil, für jeweils geradkettiges oder verzweigtes Halogenalkenyl oder Halogenalkinyl mit jeweils 3 bis 7 Kohlenstoffatomen und jeweils 1 bis 3 gleichen oder verschiedenen Halogenatomen, insbesondere Fluor, Chlor oder Brom oder für Dioxolanylalkyl, Dithio lanylalkyl, Dioxanylalkyl oder Dithianylalkyl mit jeweils 1 bis 3 Kohlenstoffatomen im geradkettigen oder verzweigten Alkylteil steht;

oder für jeweils unsubstituiertes oder ein- bis fünffach, gleich oder verschieden im Cycloalkylteil durch Fluor, Chlor, Brom oder Methyl substituiertes Cycloalkyl oder Cycloalkylalkyl mit jeweils 3 bis 6 Kohlenstoffatomen im Cycloalkylteil und 1 bis 4 Kohlenstoffatomen im geradkettigen oder verzweigten Alkylteil steht;

oder für jeweils unsubstituiertes oder ein- bis dreifach, gleich oder verschieden substituiertes Phenyl, α-Naphthyl oder ß-Naphthyl oder für einen Rest der Formel

$$Ar-(X)_m-CH_2-;\quad Ar-(X)_m-\underset{}{\overset{CH_3}{\overset{|}{C}H}}-\quad \text{oder}\quad Ar-(X)_m-\underset{\underset{CH_3}{|}}{\overset{\overset{CH_3}{|}}{C}}-\quad \text{steht,}$$

wobei

Ar jeweils für unsubstituiertes oder ein- bis dreifach, gleich oder verschieden substituiertes Phenyl, α-Naphthyl oder ß-Naphthyl steht,

X für Sauerstoff, Schwefel, Sulfinyl, Sulfonyl oder für eine der Gruppen $-CH_2-$; $-O-CH_2$; $-CH_2-O-$; $-O-CH_2-CH_2-$; $-S(O)_n-CH_2-$; $-CH_2-S(O)_n$-oder $-S(O)_n-CH_2-CH_2-$ steht,

m jeweils für eine Zahl 0 oder 1 steht und

n jeweils für eine Zahl 0, 1 oder 2 steht,

wobei als Phenylsubstituenten jeweils infrage kommen: Fluor, Chlor, Brom, Cyano, Nitro, Methyl, Ethyl, n-oder i-Propyl, n-, i-, s- oder t-Butyl, Methoxy, Ethoxy, n- oder i-Propoxy, Methylthio, Ethylthio, Propan-1,3-diyl, Butan-1,4-diyl, Pentan-1,5-diyl, Dimethylpropan-1,3-diyl, Tetramethylpropan-1,3-diyl, Dimethyl-butan-1,4-diyl, Tetramethylbutan-1,4-diyl, Cyclopropyl, Cyclobutyl, Cyclopentyl, Cylohexyl, Trifluormethyl, Dichlorfluormethyl, Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethoxy, Difluormethoxy, Fluormethoxy, Dichlorfluormethoxy, Difluorchlormethoxy, Difluorbrommethoxy, Trichlormethoxy, Trifluorethoxy, Tetrafluorethoxy,

Pentafluorethoxy, Trifluorchlorethoxy, Trifluordichlorethoxy, Difluortrichlorethoxy, Pentachlorethoxy, Trifluormethylthio, Difluormethylthio, Fluormethylthio, Difluorchlormethylthio, Dichlorfluormethylthio, Difluorethylthio, Difluorbrommethylthio, Trichlormethylthio, Trifluorethylthio, Tetrafluorethylthio, Pentafluorethylthio, Trifluorchlorethylthio, Trifluordichlorethylthio, Pentachlorethylthio, Methylsulfinyl, Trifluormethylsulfinyl, Dichlorfluormethylsulfinyl, Difluorchlormethylsulfinyl, Fluormethylsulfinyl, Difluormethylsulfinyl, Methylsulfonyl, Trifluormethylsulfonyl, Dichlorfluormethylsulfonyl, Difluorchlormethyl sulfonyl, Fluormethylsulfonyl, Difluormethylsulfonyl, Methoxycarbonyl, Ethoxycarbonyl, Methoximinomethyl, Ethoximinomethyl, Methoximinoethyl, Ethoximinoethyl, Difluordioxymethylen, Tetrafluordioxyethylen, Trifluordioxyethylen, Difluordioxyethylen, Dioxymethylen, Dioxyethylen, unsubstituiertes oder ein- bis dreifach durch Fluor oder Chlor substituiertes Phenyl oder Phenoxy;

und wobei als Naphthylsubstituenten jeweils infrage kommen: Fluor, Chlor, Brom, Cyano, Methyl, Ethyl, n- oder i-Propyl,

$R^2$, $R^3$, $R^4$, $R^5$ und $R^6$ unabhängig voneinander für Wasserstoff oder Methyl stehen und

A für Stickstoff oder eine CH-Gruppe steht.

Insbesondere bevorzugt sind Verbindungen der Formel (I), in denen

$R^1$ für jeweils geradkettiges oder verzweigtes Alkyl mit 3 bis 7 Kohlenstoffatomen, Alkenyl mit 3 bis 7 Kohlenstoffatomen oder Alkinyl mit 3 bis 7 Kohlenstoffatomen steht;

oder für jeweils ein- bis dreifach, gleich oder verschieden durch Fluor, Chlor, Methoxy, Ethoxy, Methylthio, Ethylthio, Trifluormethyl, Trifluormethoxy oder Trifluormethylthio substituiertes i-Propyl oder t-Butyl steht;

oder für jeweils einfach durch Cyano, Hydroximino, Methoximino, Ethoximino, Cyclopentyl, Cyclohexyl, Dioxolanyl, Dithiolanyl, Dioxanyl oder Dithianyl substituiertes Methyl, Ethyl, i-Propyl oder t-Butyl steht;

oder für jeweils geradkettiges oder verzweigtes, einfach oder zweifach, gleich oder verschieden durch Fluor, Chlor oder Brom substituiertes Alkenyl oder Alkinyl mit jeweils 3 bis 7 Kohlenstoffatomen steht;

oder für jeweils unsubstituiertes oder ein- bis fünffach, gleich oder verschieden durch Fluor, Chlor, Brom oder Methyl substituiertes Cyclopropyl, Cyclobutyl, Cyclopentyl oder Cyclohexyl steht; oder für α-Naphthyl oder ß-Naphthyl steht; oder für unsubstituiertes oder ein- bis dreifach, gleich oder verschieden durch Fluor, Chlor, Brom, Methyl, Methoxy, Methylthio, Trifluormethyl, Trifluormethoxy, Trifluormethylthio, Difluormethyl, Difluormethoxy, Difluormethylthio, Phenyl oder Phenoxy substituiertes Phenyl steht;

oder für einen Rest der Formel

$$Ar-(X)_m-CH_2-;\quad Ar-(X)_m-\overset{\overset{\displaystyle CH_3}{|}}{CH}-\quad \text{oder}\quad Ar-(X)_m-\underset{\underset{\displaystyle CH_3}{|}}{\overset{\overset{\displaystyle CH_3}{|}}{C}}-\quad \text{steht,}$$

wobei

Ar jeweils für unsubstituieres oder ein- bis dreifach, gleich oder verschieden durch Fluor, Chlor, Brom, Methyl, Methoxy, Methylthio, Trifluormethyl, Trifluormethoxy, Trifluormethylthio, Difluormethyl, Difluormethoxy, Difluormethylthio, Phenyl oder Phenoxy substituiertes Phenyl steht oder für jeweils unsubstituiertes oder durch Fluor, Chlor oder Methyl substituiertes α-Naphthyl oder ß-Naphthyl steht,

X für Sauerstoff, Schwefel oder für eine der Gruppen $-CH_2-$, $-O-CH_2-$, $-S-CH_2-$, $-O-CH_2-CH_2-$ oder $-S-CH_2-CH_2-$ steht und

m für eine Zahl 0 oder 1 steht,

$R^2$, $R^3$, $R^4$, $R^5$ und $R^6$ für Wasserstoff stehen und

A für Stickstoff oder eine CH-Gruppe steht.

Bevorzugte erfindungsgemäße Verbindungen sind auch Additionsprodukte aus Säuren und denjenigen substituierten Dioxolanen der Formel (I), in denen die Substituenten $R^1$, $R^2$, $R^3$, $R^4$, $R^5$, $R^6$ und A die Bedeutungen haben, die bereits vorzugsweise für diese Substituenten genannt wurden, insbesondere die pflanzenverträglichen Säureadditionssalze.

Zu den Säuren, die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie z.B. p-Toluolsulfonsäure und 1,5-Naphthalindisulfonsäure oder Saccharin oder Thiosaccharin.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen substituierten Dioxolanen der Formel (I), in denen die Substituenten $R^1$, $R^2$, $R^3$, $R^4$, $R^5$, $R^6$ und A die Bedeutungen haben, die bereits vorzugsweise für diese Substituenten genannt wurden, insbesondere die pflanzenverträglichen Metallkomplexe.

Hierbei sind die Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu pflanzenverträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, Salpetersäure und Schwefelsäure.

Im einzelnen seien außer den bei den Herstellungsbeispielen genannten Verbindungen die folgenden substituierten Dioxolane der allgemeinen Formel (I) genannt:

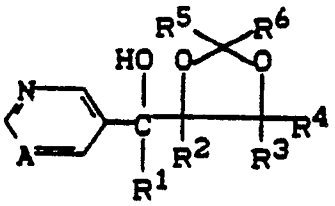

(I)

| $R^1$ | 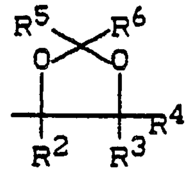 | A |
|---|---|---|
| 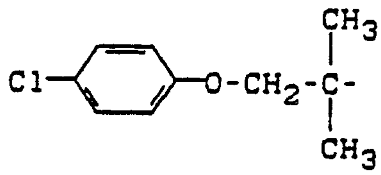 | 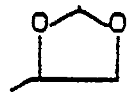 | N |
| 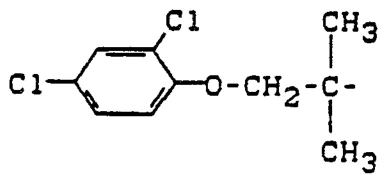 | 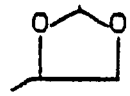 | N |
| 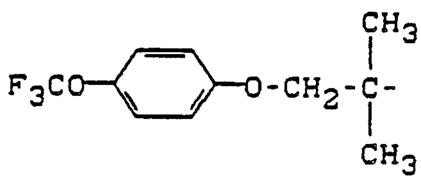 | 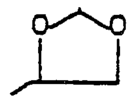 | N |
| 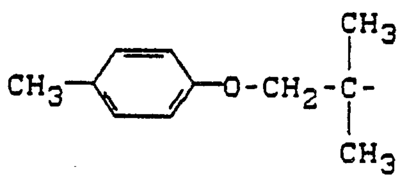 | 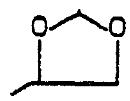 | N |
| 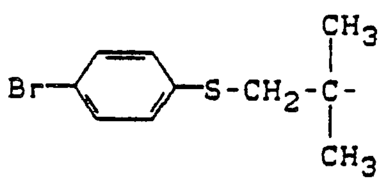 | 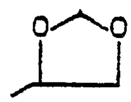 | N |
| 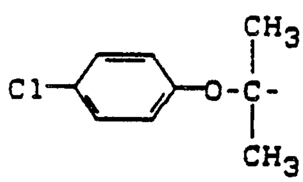 | 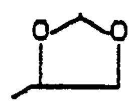 | N |
| 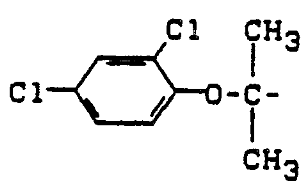 | 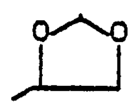 | N |

| $R^1$ | 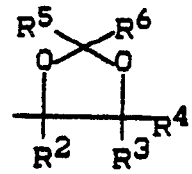 | A |
|---|---|---|
| 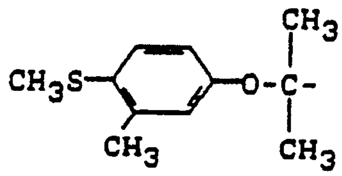 | 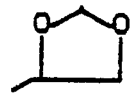 | N |
| 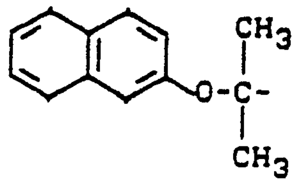 | 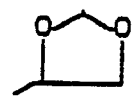 | N |
| 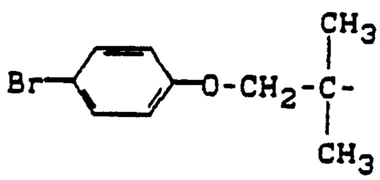 | 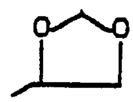 | N |
| 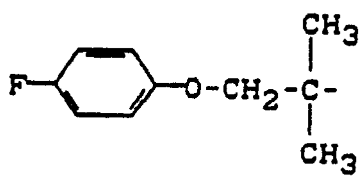 | 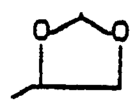 | N |
| 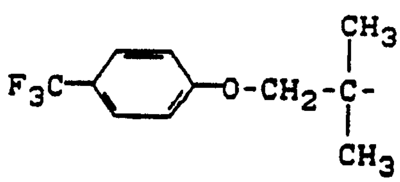 | 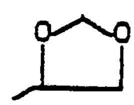 | N |
| 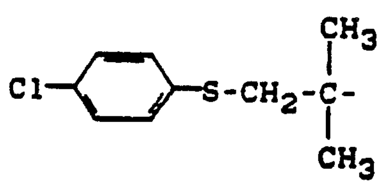 | 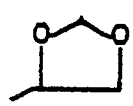 | N |
| 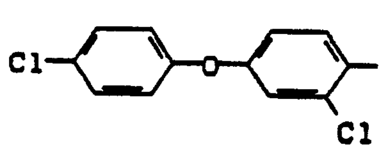 | 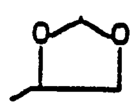 | N |

| $R^1$ | 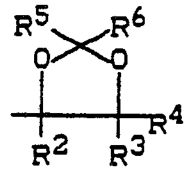 | A |
|---|---|---|
| 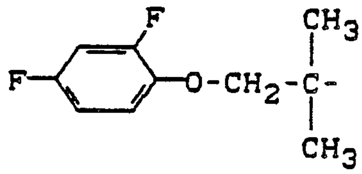 | 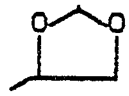 | N |
| 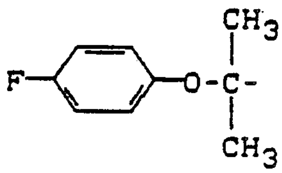 | 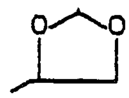 | N |
| 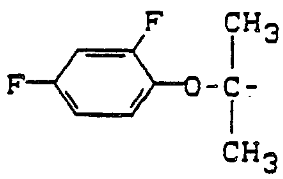 | 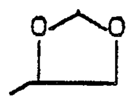 | N |
| 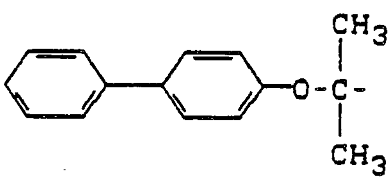 | 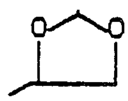 | N |
| 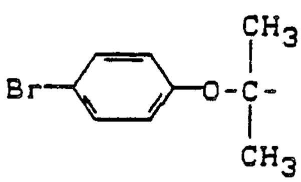 | 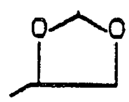 | N |
| 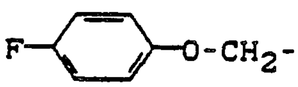 | 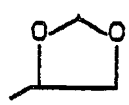 | N |
| 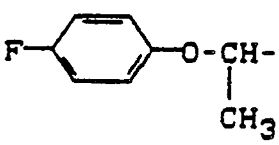 | 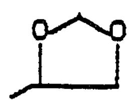 | N |

| $R^1$ | 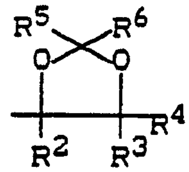 | A |
|---|---|---|
| 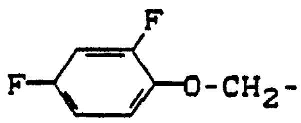 | 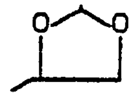 | N |
| 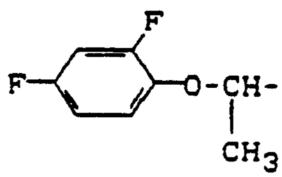 | 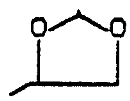 | N |
| 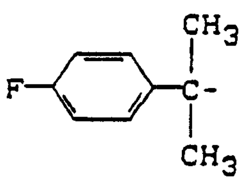 | 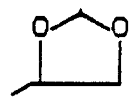 | N |
| 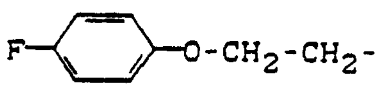 | 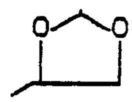 | N |
| 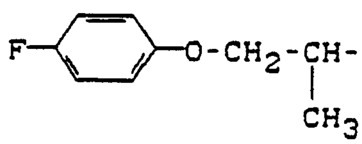 | 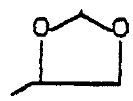 | N |
| 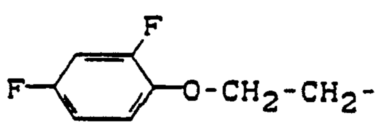 | 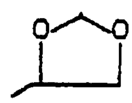 | N |
| 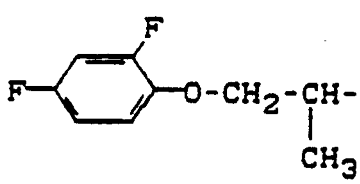 | 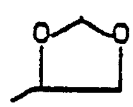 | N |

| $R^1$ | 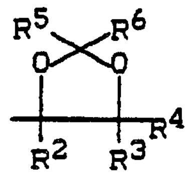 | A |
|---|---|---|
| 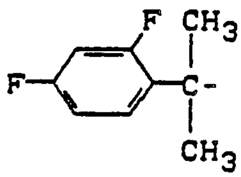 | 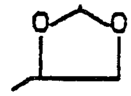 | N |
| 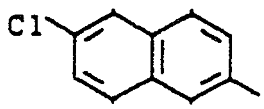 | 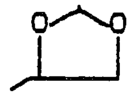 | N |
| 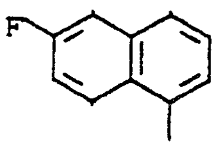 | 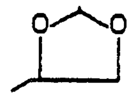 | N |
| 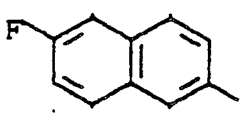 | 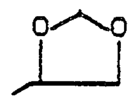 | N |
| 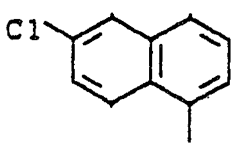 | 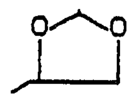 | N |
| 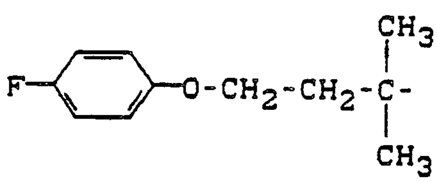 | 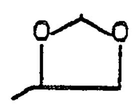 | N |
| 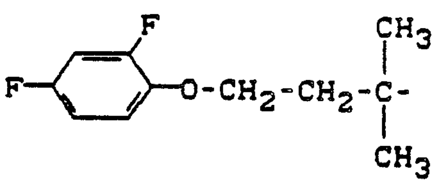 | 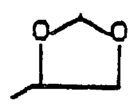 | N |
| 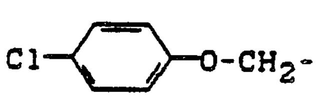 | 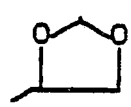 | N |

| $R^1$ | 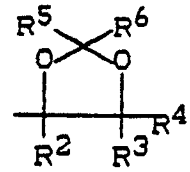 | A |
| --- | --- | --- |
| 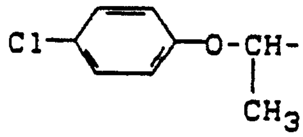 | 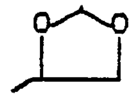 | N |
| 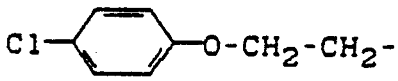 | 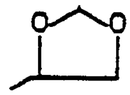 | N |
| 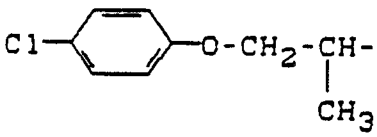 | 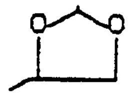 | N |
| 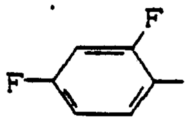 | 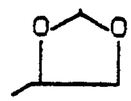 | N |
| 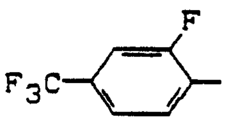 | 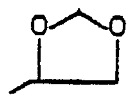 | N |
| 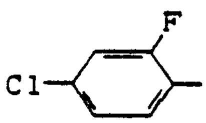 | 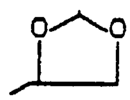 | N |
| 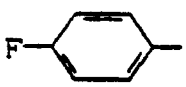 | 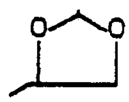 | N |
| 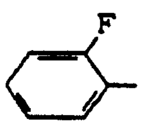 | 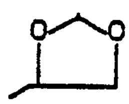 | N |

| $R^1$ | 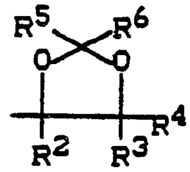 | A |
|---|---|---|
| 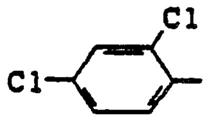 | 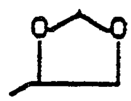 | N |
| 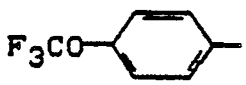 | 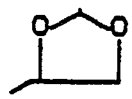 | N |
| 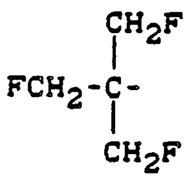 | 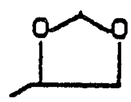 | N |
| 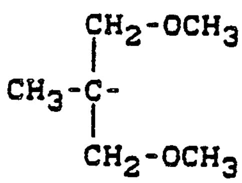 | 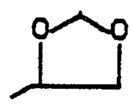 | N |
| 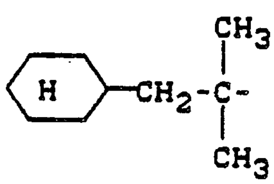 | 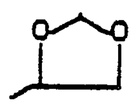 | N |
| 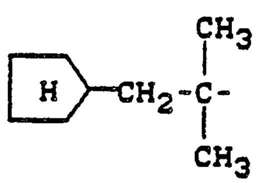 | 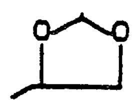 | N |
| 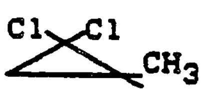 | 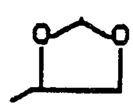 | N |
| 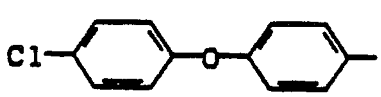 | 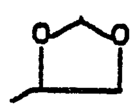 | N |

| $R^1$ | 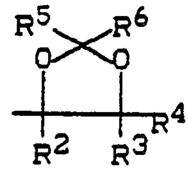 | A |
|---|---|---|
| 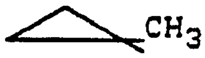 | 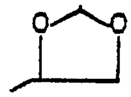 | N |
| 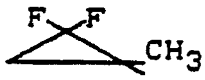 | 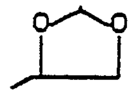 | N |
| 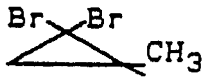 | 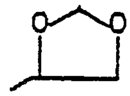 | N |
| 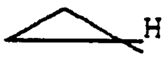 | 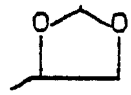 | N |
| 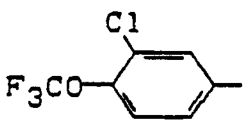 | 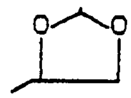 | N |
| 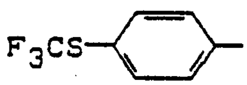 | 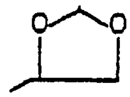 | N |
| 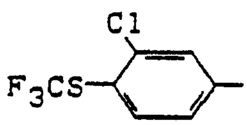 | 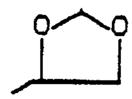 | N |
| 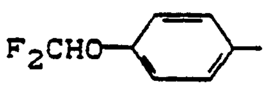 | 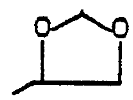 | N |
| 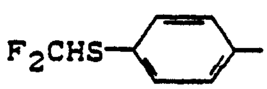 | 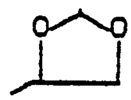 | N |

| $R^1$ | 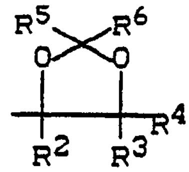 | A |
|---|---|---|
| 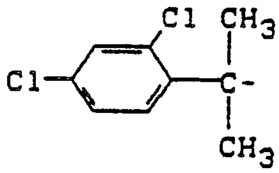 | 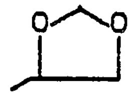 | N |
| 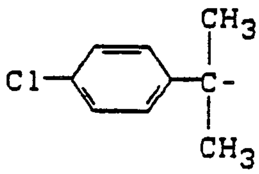 | 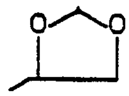 | N |
| 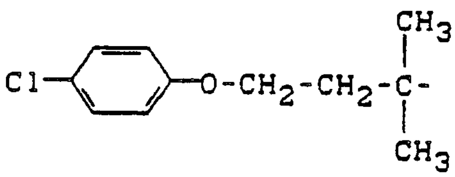 | 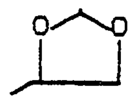 | N |
| 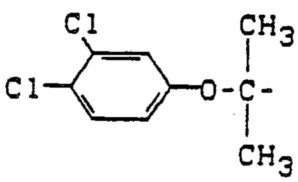 | 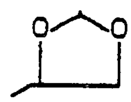 | N |
| 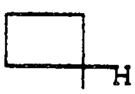 | 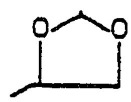 | N |
| 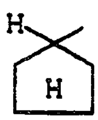 | 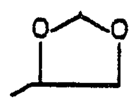 | N |
| 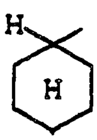 | 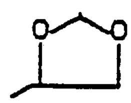 | N |

| $R^1$ | 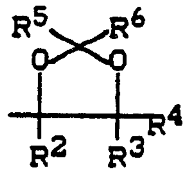 | A |
|---|---|---|
| 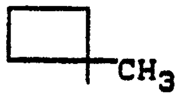 | 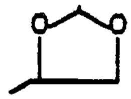 | N |
| 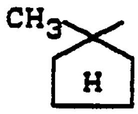 | 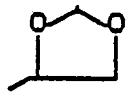 | N |
| 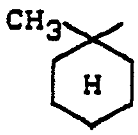 | 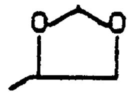 | N |
| 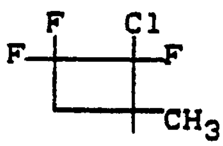 | 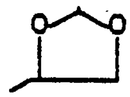 | N |
| $ClCH=CH-CH_2-$ | 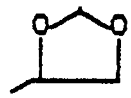 | N |
| $(CH_3)_3C-$ | 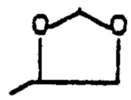 | N |
| $(CH_3)_2CH-$ | 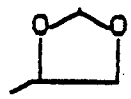 | N |
| 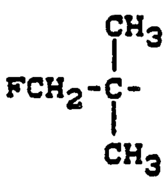 | 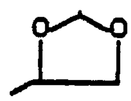 | N |
| 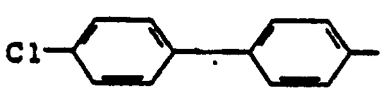 | 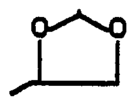 | N |

| $R^1$ | 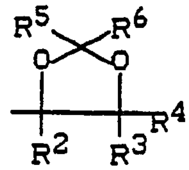 | A |
|---|---|---|
| 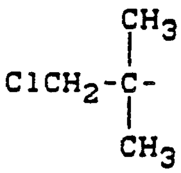 | 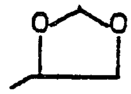 | N |
| 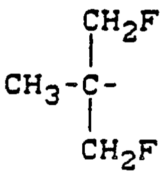 | 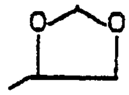 | N |
| 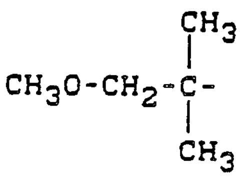 | 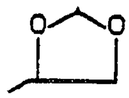 | N |
| 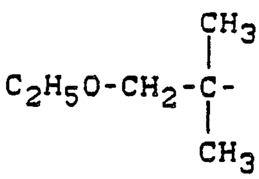 | 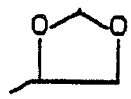 | N |
| 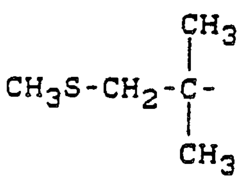 | 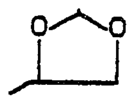 | N |
| 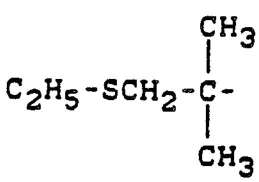 | 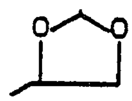 | N |

| $R^1$ | 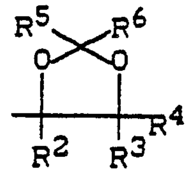 | A |
|---|---|---|
| 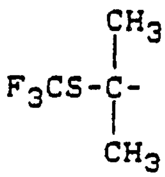 | 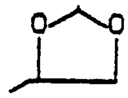 | N |
| 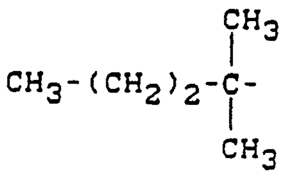 | 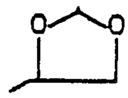 | N |
| 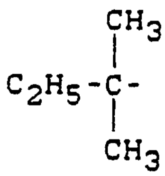 | 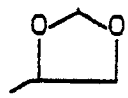 | N |
| 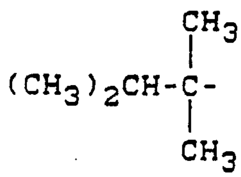 | 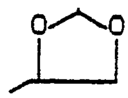 | N |
| 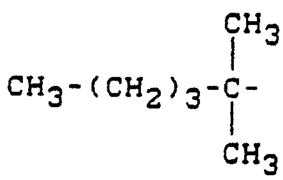 | 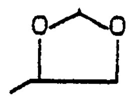 | N |
| 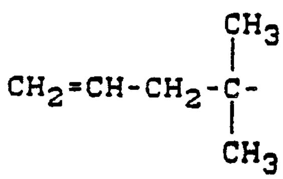 | 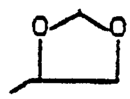 | N |
| 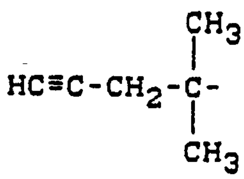 | 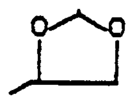 | N |

| $R^1$ | 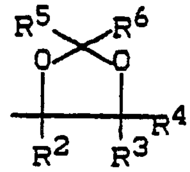 | A |
|---|---|---|
| 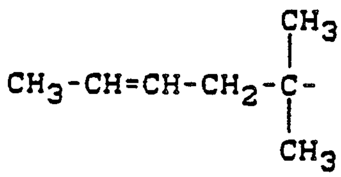 | 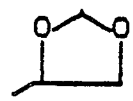 | N |
| 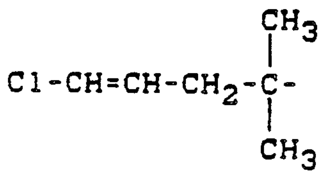 | 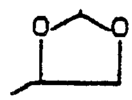 | N |
| 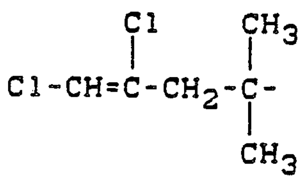 | 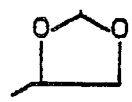 | N |
| 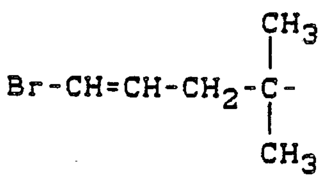 | 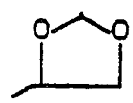 | N |
| 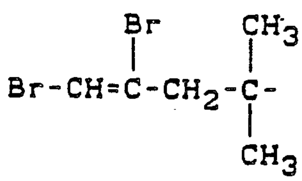 | 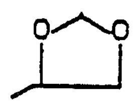 | N |
| $H_2C=CH-CH_2-$ | 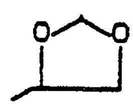 | N |
| $HC\equiv C-CH_2-$ | 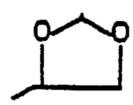 | N |

| $R^1$ | 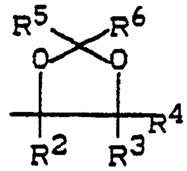 | A |
|---|---|---|
| 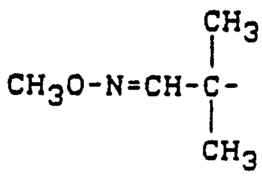 | 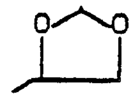 | N |
| 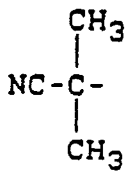 | 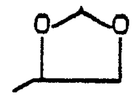 | N |
| 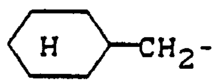 | 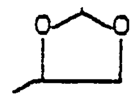 | N |
| 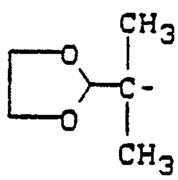 | 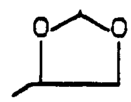 | N |
| 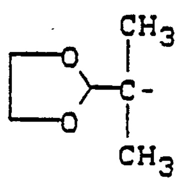 | 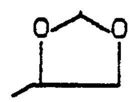 | N |
| 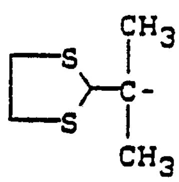 | 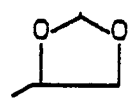 | N |
| 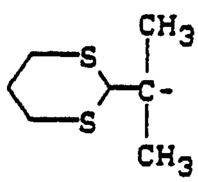 | 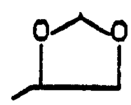 | N |

| $R^1$ | 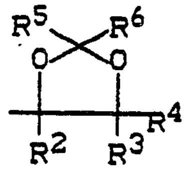 | A |
|---|---|---|
| 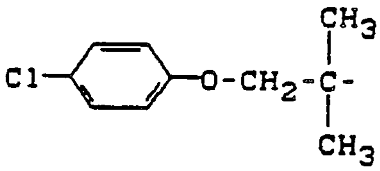 | 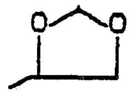 | CH |
| 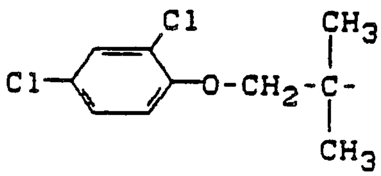 | 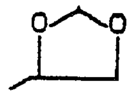 | CH |
| 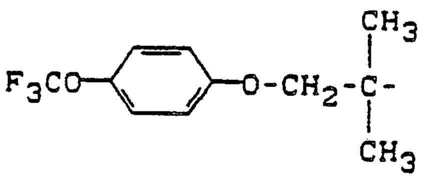 | 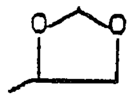 | CH |
| 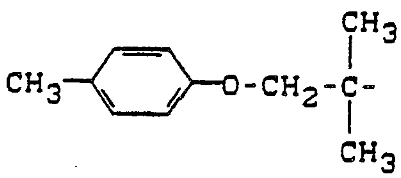 | 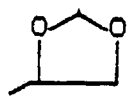 | CH |
| 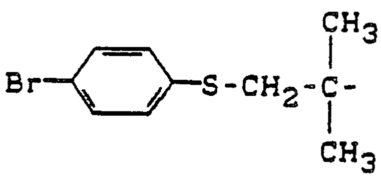 | 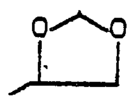 | CH |
| 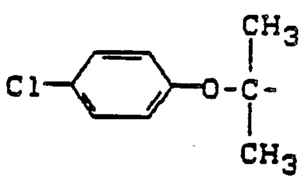 | 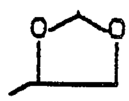 | CH |
| 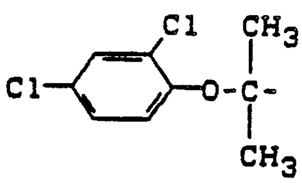 | 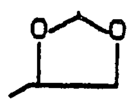 | CH |

| $R^1$ | 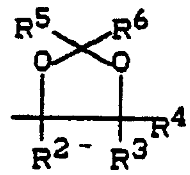 | A |
|---|---|---|
| 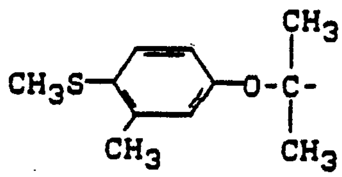 | 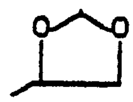 | CH |
| 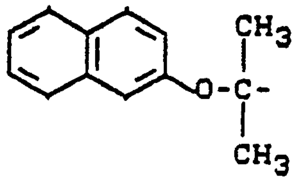 | 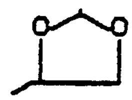 | CH |
| 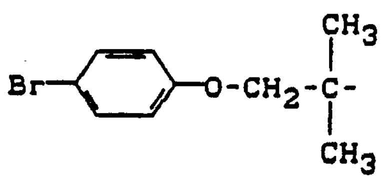 | 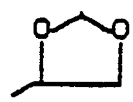 | CH |
| 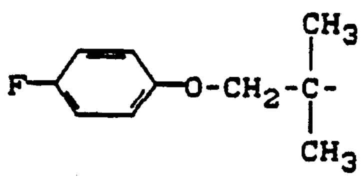 | 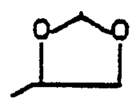 | CH |
| 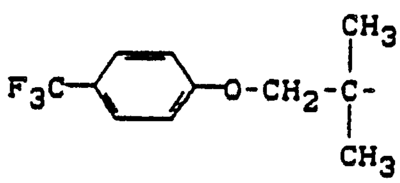 | 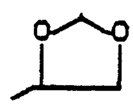 | CH |
| 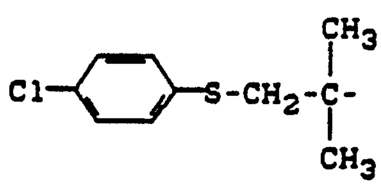 | 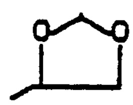 | CH |
| 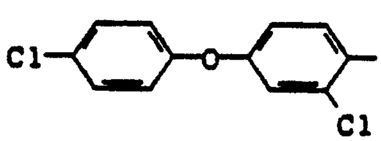 | 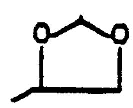 | CH |

| $R^1$ | 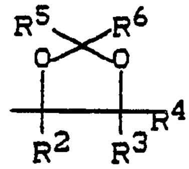 | A |
|---|---|---|
| 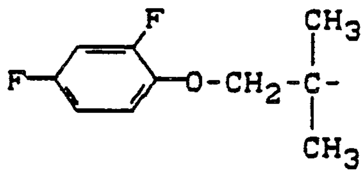 | 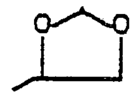 | CH |
| 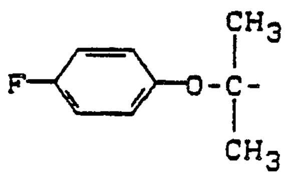 | 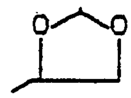 | CH |
| 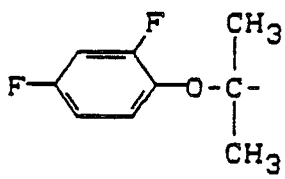 | 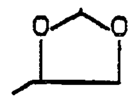 | CH |
| 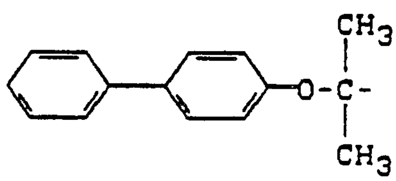 | 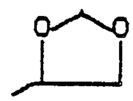 | CH |
| 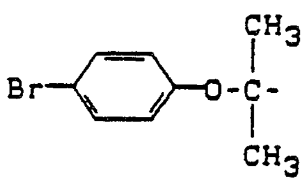 | 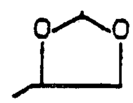 | CH |
| 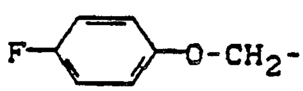 | 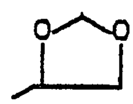 | CH |
| 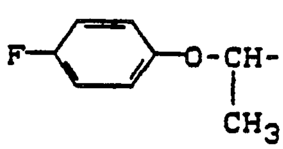 | 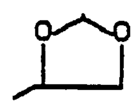 | CH |

| $R^1$ | 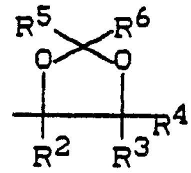 | A |
|---|---|---|
| 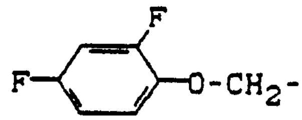 | 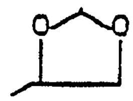 | CH |
| 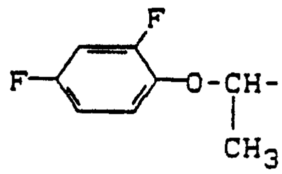 | 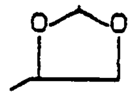 | CH |
| 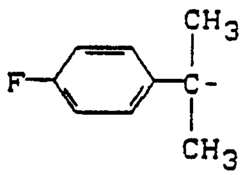 | 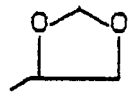 | CH |
| 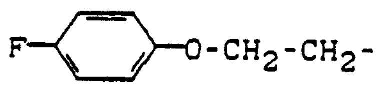 | 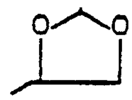 | CH |
| 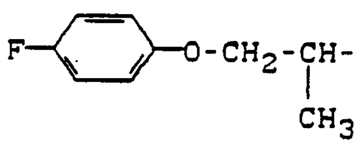 | 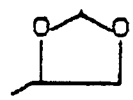 | CH |
| 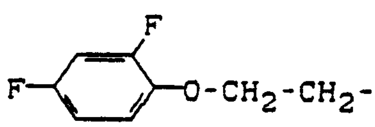 | 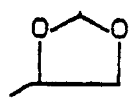 | CH |
| 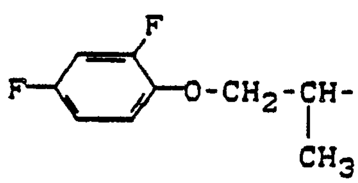 | 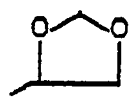 | CH |

| $R^1$ | 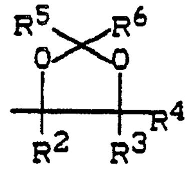 | A |
|---|---|---|
| 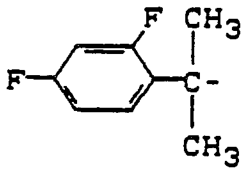 | 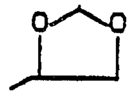 | CH |
| 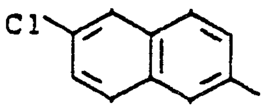 | 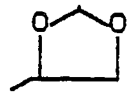 | CH |
| 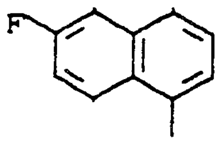 | 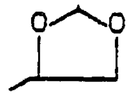 | CH |
| 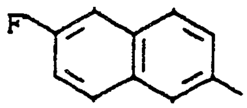 | 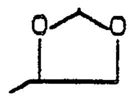 | CH |
| 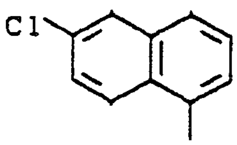 | 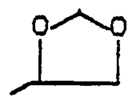 | CH |
| 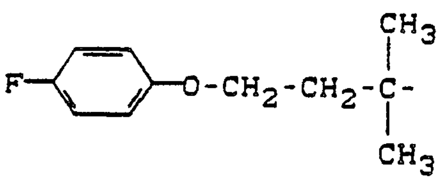 | 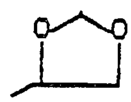 | CH |
| 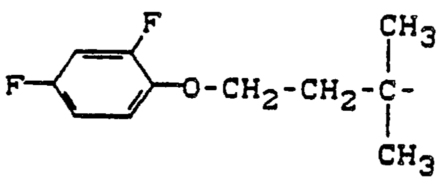 | 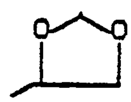 | CH |
| 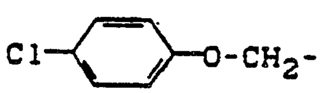 | 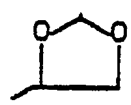 | CH |

| $R^1$ | 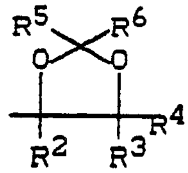 | A |
|---|---|---|
| 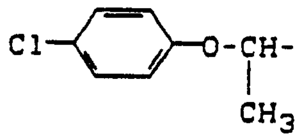 | 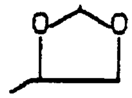 | CH |
| 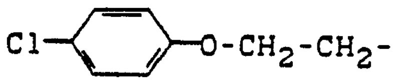 | 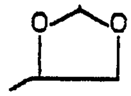 | CH |
| 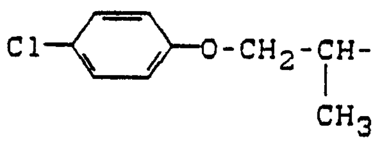 | 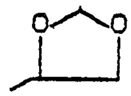 | CH |
| 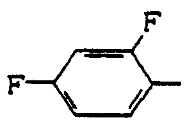 | 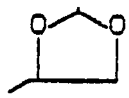 | CH |
| 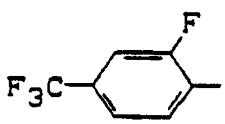 | 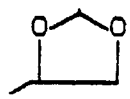 | CH |
| 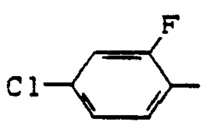 | 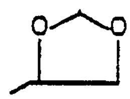 | CH |
| 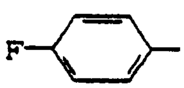 | 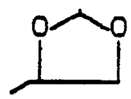 | CH |
| 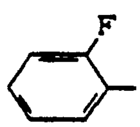 | 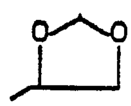 | CH |

| $R^1$ | 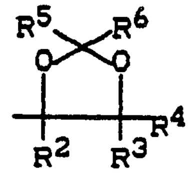 | A |
|---|---|---|
| 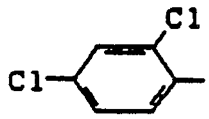 | 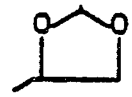 | CH |
| 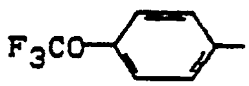 | 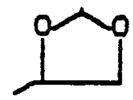 | CH |
| 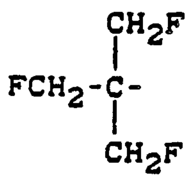 | 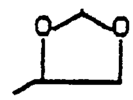 | CH |
| 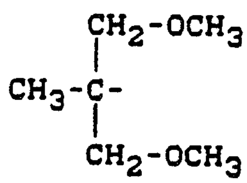 | 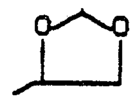 | CH |
| 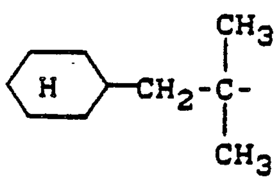 | 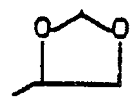 | CH |
| 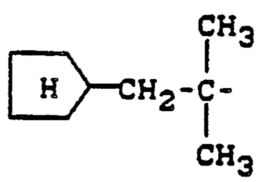 | 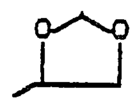 | CH |
| 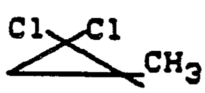 | 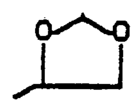 | CH |
| 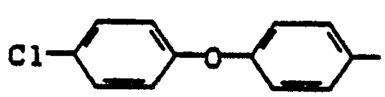 | 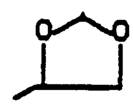 | CH |

| R¹ | 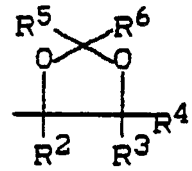 | A |
|---|---|---|
| 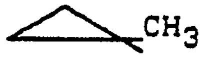 | 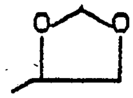 | CH |
| 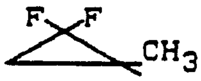 | 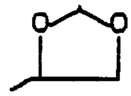 | CH |
| 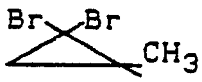 | 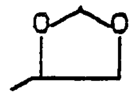 | CH |
| 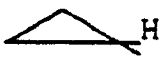 | 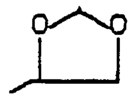 | CH |
| 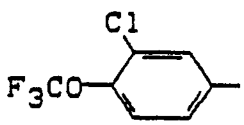 | 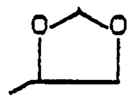 | CH |
| 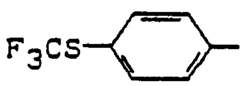 | 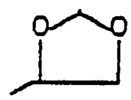 | CH |
| 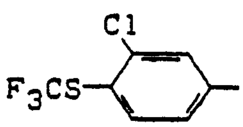 | 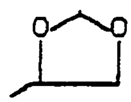 | CH |
| 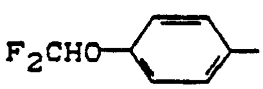 | 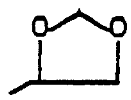 | CH |
| 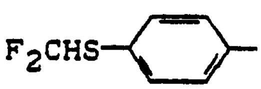 | 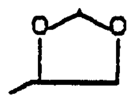 | CH |

| $R^1$ | 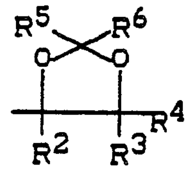 | A |
|---|---|---|
| 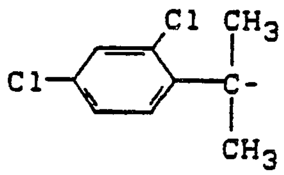 | 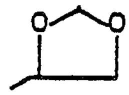 | CH |
| 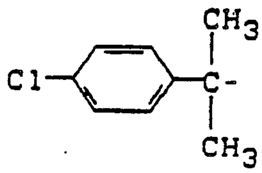 | 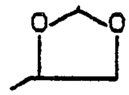 | CH |
| 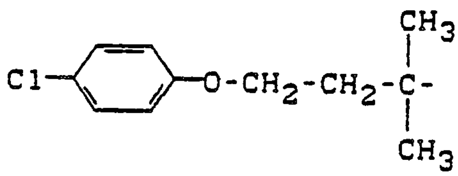 | 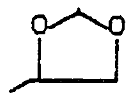 | CH |
| 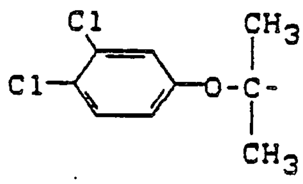 | 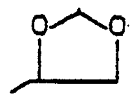 | CH |
| 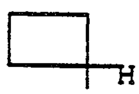 | 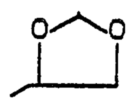 | CH |
| 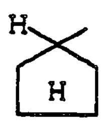 | 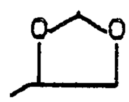 | CH |
| 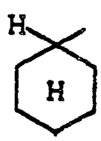 | 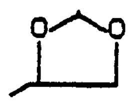 | CH |

| $R^1$ | 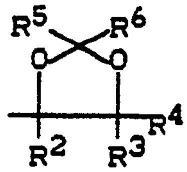 | A |
|---|---|---|
| 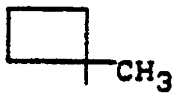 | 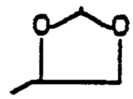 | CH |
| 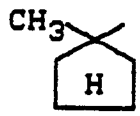 | 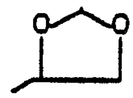 | CH |
| 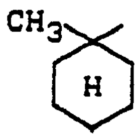 | 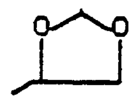 | CH |
| 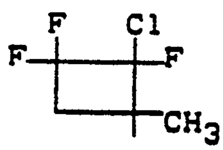 | 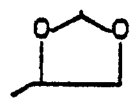 | CH |
| $ClCH=CH-CH_2-$ | 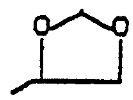 | CH |
| $(CH_3)_3C-$ | 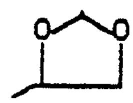 | CH |
| $(CH_3)_2CH-$ | 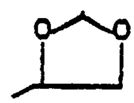 | CH |
| 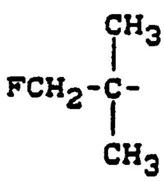 | 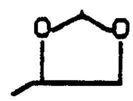 | CH |
| 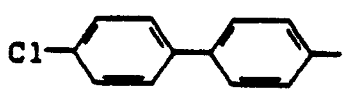 | 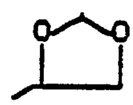 | CH |

| $R^1$ | 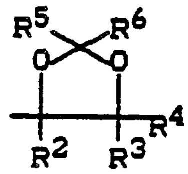 | A |
|---|---|---|
| 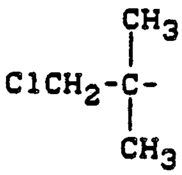 | 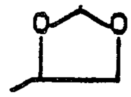 | CH |
| 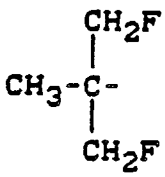 | 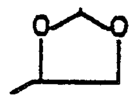 | CH |
| 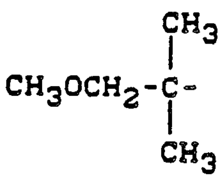 | 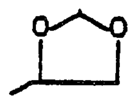 | CH |
| 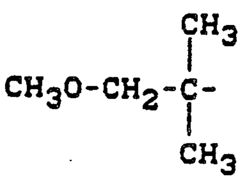 | 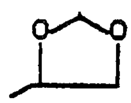 | CH |
| 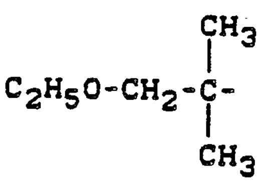 | 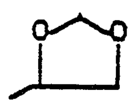 | CH |
| 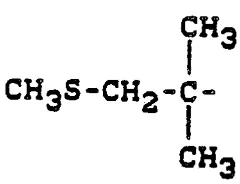 | 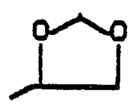 | CH |
| 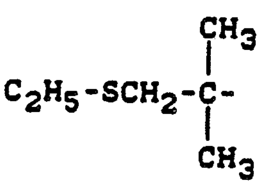 | 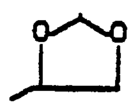 | CH |

| $R^1$ | 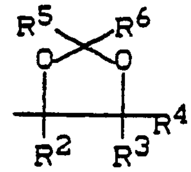 | A |
|---|---|---|
| 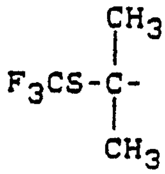 | 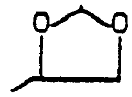 | CH |
| 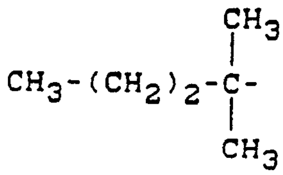 | 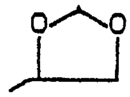 | CH |
| 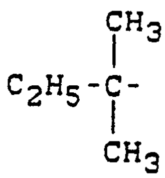 | 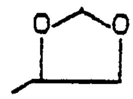 | CH |
| 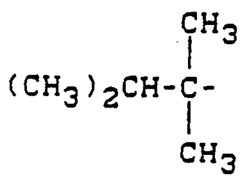 | 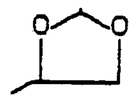 | CH |
| 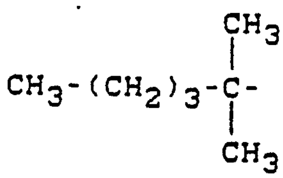 | 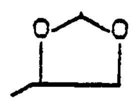 | CH |
| 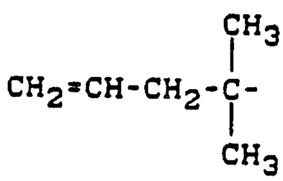 | 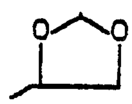 | CH |
| 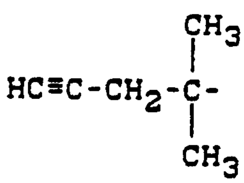 | 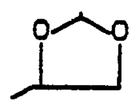 | CH |

| $R^1$ | 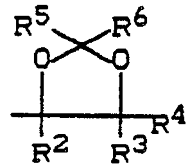 | A |
| --- | --- | --- |
| 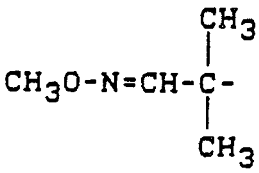 | 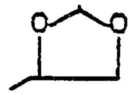 | CH |
| 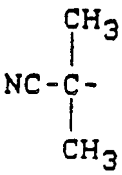 | 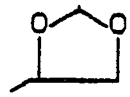 | CH |
| 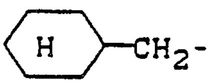 | 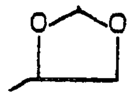 | CH |
| 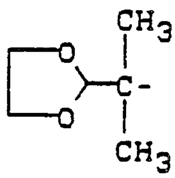 | 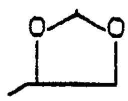 | CH |
| 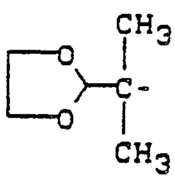 | 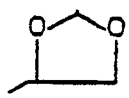 | CH |
| 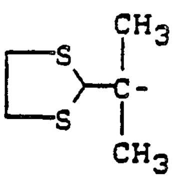 | 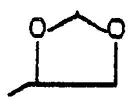 | CH |
| 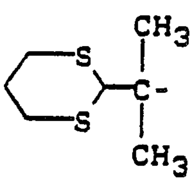 | 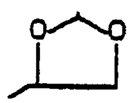 | CH |

Verwendet man beispielsweise 4-(4-Chlorbenzoyl)-1,3-dioxolan und Pyridylmagnesiumbromid als Ausgangsstoffe, so läßt sich der Reaktionsablauf des erfindungsgemäßen Verfahrens durch das folgende Formelschema darstellen:

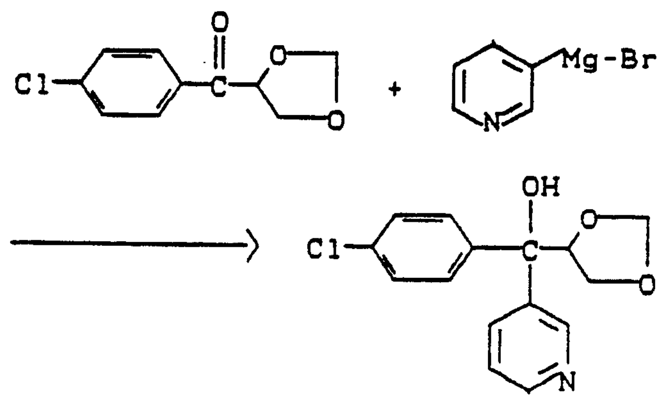

Die zur Durchführung des erfindungsgemäßen Verfahrens als Ausgangsstoffe benötigten Dioxolanylketone sind durch die Formel (II) allgemein definiert. In dieser Formel (II) stehen $R^1$, $R^2$, $R^3$, $R^4$, $R^5$ und $R^6$ vorzugsweise für diejenigen Reste, die bereits im Zusammenhang mit der Beschreibung der erfindungsgemäßen Stoffe der Formel (I) als bevorzugt für diese Substituenten genannt wurden.

Die Dioxolanylketone der Formel (II) sind teilweise bekannt (vgl. z.B. J. org. Chem. 33, 2473-2477 [1968]; Acta chem. Scand. B 34, 41-45 [1980]; Tetrahedron 36, 3101-3105 [1980]; J. org. Chem. 47, 3289-3296 [1982]; Tetrahedron Lett. 23, 4369-4370 [1982]; Synthesis 1986 60-61; Synth. Commun. 16, 1517-1522 [1986]; Tetrahedron Lett. 28, 383-386 [1987]).

Noch nicht bekannt und Gegenstand einer parallelen Anmeldung sind Dioxolanylketone der Formel (IIa),

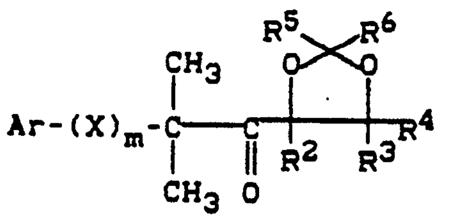

(IIa)

in welcher
Ar für gegebenenfalls substituiertes Aryl steht,
X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$ oder $-S(O)_n-CH_2-CH_2-$ steht,
m für eine Zahl 0 oder 1 steht,
n jeweils für eine Zahl 0, 1 oder 2 steht und
$R^2$, $R^3$, $R^4$, $R^5$ und $R^6$ die oben angegebene Bedeutung haben.

Bevorzugt sind Dioxolanylketone der Formel (IIa), in denen
Ar für unsubstituierte oder einfach bis mehrfach, gleich oder verschieden substituiertes Phenyl steht, wobei als Substituenten infrage kommen: Halogen, Cyano,
Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, geradkettiges oder verzweigtes, zweifach verknüpftes Alkandiyl mit 1 bis 10 Kohlenstoffatomen, Cycloalkyl mit 3 bis 7 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, unsubstituiertes oder einfach bis mehrfach, gleich oder verschieden durch Halogen substituiertes, zweifach verknüpftes Dioxyalkylen mit 1 bis 6 Kohlenstoffatomen oder jeweils unsubstituiertes oder einfach bis mehrfach, gleich oder verschieden durch Halogen substituiertes Phenyl oder Phenoxy;
oder für unsubstituiertes oder einfach bis mehrfach, gleich oder verschieden substituiertes Naphthyl steht, wobei als Substituenten infrage kommen: Halogen, Cyano oder geradkettiges oder verzweigtes Alkyl mit 1 bis 4 Kohlenstoffatomen;
X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$ oder $-S(O)_n-CH_2-CH_2-$ steht,
m für eine Zahl 0 oder 1 steht,

n jeweils für eine Zahl 0, 1 oder 2 steht und
$R^2$, $R^3$, $R^4$, $R^5$ und $R^6$ die oben angegebene Bedeutung haben.

Besonders bevorzugt sind Dioxolanylketone der Formel (IIa), in denen
Ar für unsubstituiertes oder ein- bis dreifach, gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Propan-1,3-diyl, Butan-1,4-diyl, Pentan-1,5-diyl, Dimethylpropan-1,3-diyl, Tetramethylpropan-1,3-diyl, Dimethylbutan-1,4-diyl, Tetramethylbutan-1,4-diyl, Cyclopropyl, Cyclobutyl, Cyclopentyl, Cylohexyl, Trifluormethyl, Dichlorfluormethyl, Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethoxy, Difluormethoxy, Fluormethoxy, Dichlorfluormethoxy, Difluorchlormethoxy, Difluorbrommethoxy, Trichlormethoxy, Trifluorethoxy, Tetrafluorethoxy, Pentafluorethoxy, Trifluorchlorethoxy, Trifluordichlorethoxy, Difluortrichlorethoxy, Pentachlorethoxy, Trifluormethylthio, Difluormethylthio, Fluormethylthio, Difluorchlormethylthio, Dichlorfluormethylthio, Difluorethylthio, Difluorbrommethylthio, Trichlormethylthio, Trifluorethylthio, Tetrafluorethylthio, Pentafluorethylthio, Trifluorchlorethylthio, Trifluordichlorethylthio, Pentachlorethylthio, Methylsulfinyl, Trifluormethylsulfinyl, Dichlorfluormethylsulfinyl, Difluorchlormethylsulfinyl, Fluormethylsulfinyl, Difluormethylsulfinyl, Methylsulfonyl, Trifluormethylsulfonyl, Dichlorfluormethylsulfonyl, Difluorchlormethylsulfonyl, Fluormethylsulfonyl, Difluormethylsulfonyl, Methoxycarbonyl, Ethoxycarbonyl, Methoximinomethyl, Ethoximinomethyl, Methoximinoethyl, Ethoximinoethyl, Difluordioxymethylen, Tetrafluordioxyethylen, Trifluordioxyethylen, Difluordioxyethylen, Dioxymethylen, Dioxyethylen, jeweils unsubstituiertes oder ein- bis dreifach, gleich oder verschieden durch Fluor oder Chlor substituiertes Phenyl oder Phenoxy;
oder für unsubstituiertes oder ein- bis dreifach, gleich oder verschieden substituiertes α-Naphthyl oder β-Naphthyl steht, wobei als Substituenten jeweils infrage kommen: Fluor, Chlor, Brom, Cyano, Methyl, Ethyl, n- oder i-Propyl;
X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$ oder $-S(O)_n-CH_2-CH_2-$ steht,
m für eine Zahl 0 oder 1 steht,
n jeweils für eine Zahl 0, 1 oder 2 steht und
$R^2$, $R^3$, $R^4$, $R^5$ und $R^6$ die oben angegebene Bedeutung haben.

Ganz besonders bevorzugt sind Dioxolanylketone der Formel (IIa), in denen
Ar für unsubstituiertes oder ein- bis dreifach, gleich oder verschieden durch Fluor, Chlor, Brom, Methyl, Methoxy, Methylthio, Trifluormethyl, Trifluormethoxy, Trifluormethylthio, Difluormethyl, Difluormethoxy, Difluormethylthio, Phenyl oder Phenoxy substituiertes Phenyl steht;
oder für jeweils unsubstituiertes oder durch Fluor, Chlor oder Methyl substituiertes α-Naphthyl oder β-Naphthyl steht,
X für Sauerstoff, Schwefel oder für eine der Gruppen $-CH_2-$; $-O-CH_2-$; $-S-CH_2-$; $-O-CH_2-CH_2-$ oder $-S-CH_2-CH_2-$ steht,
m für eine Zahl 0 oder 1 steht und
$R^2$, $R^3$, $R^4$, $R^5$ und $R^6$ die oben angegebene Bedeutung haben.

Man erhält diese neuen Dioxolanylketone in Analogie zur Herstellung der bekannten Dioxolanylketone der Formel (II), wenn man Vinylketone der Formel (IVa),

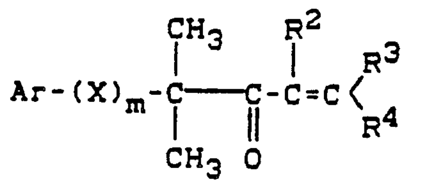

(IVa)

in welcher
Ar, X, $R^2$, $R^3$, $R^4$ und m die oben angegebene Bedeutung haben,
mit einem Oxidationsmittel wie beispielsweise Peressigsäure oder m-Chlorperbenzoesäure gegebenenfalls in Gegenwart eines Verdünnungsmittels wie beispielsweise Dichlormethan, Dichlorbenzol, Toluol oder Essigsäure bei Temperaturen zwischen 10 °C und 60 °C epoxidiert und dann die so erhältlichen Oxiranylketone der Formel (Va),

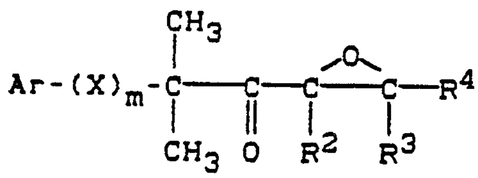 (Va)

in welcher
Ar, X, $R^2$, $R^3$, $R^4$ und m die oben angegebene Bedeutung haben,
in einer 2. Stufe mit Aldehyden oder Ketonen der Formel (VI),

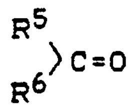 (VI)

in welcher
$R^5$ und $R^6$ die oben angegebene Bedeutung haben,
gegebenenfalls in Gegenwart eines Katalysators wie beispielsweise Zinntetrachlorid und gegebenenfalls in Gegenwart eines Verdünnungsmittels wie beispielsweise Tetrachlorkohlenstoff bei Temperaturen zwischen - 80 °C und + 50 °C umsetzt (vgl. z.B. Angew. Chem. Int. Ed. Engl. 21, 449 [1982]).

Vinylketone der Formel (IVa) sind bekannt oder erhältlich in Analogie zu bekannten Verfahren (vgl. z.B. EP 6718; DE-OS 29 22 070; Chem. Lett. 1987, 1283-1286; J. Amer. chem. Soc. 108, 4568-4580 [1986]; J. org. Chem. 51, 2389-2391 [1986] oder An. Quim. 75, 707-711 [1979] bzw. CA 92: 75 727a).

Dioxolanylketone der Formel (II) erhält man alternativ auch, wenn man Dioxolanylcarbinole der Formel (VII),

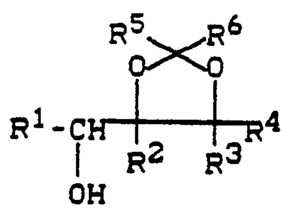 (VII)

in welcher
$R^1$, $R^2$, $R^3$, $R^4$, $R^5$ und $R^6$ die oben angegebene Bedeutung haben,
mit üblichen Oxidationsmitteln wie beispielsweise Chromtrioxid in Gegenwart von Pyridin und Chlorwasserstoff sowie gegebenenfalls in Gegenwart eines Verdünnungsmittels wie beispielsweise Dichlormethan bei Temperaturen zwischen - 20 °C und + 80 °C oxidiert (vgl. z.B. Tetrahedron Lett. 28, 383-386 [1987]).

Dioxolanylcarbinole der Formel (VII) sind bekannt oder erhältlich in Analogie zu bekannten Verfahren (vgl. z.B. Tetrahedron Lett. 26, 5759-5762 [1985]).

Dioxolanylketone der Formel (IIb),

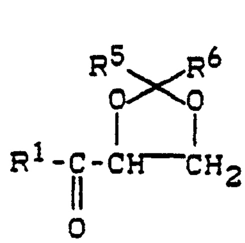 (IIb)

in welcher
$R^1$, $R^5$ und $R^6$ die oben angegebene Bedeutung haben,
erhält man alternativ auch, wenn man Halogenmethylketone der Formel (VIII),

$R^1$- C -$CH_2$-Hal (VIII)
‖
O

in welcher
$R^1$ die oben angegebene Bedeutung hat und
Hal für Halogen, insbesondere für Chlor oder Brom steht,
mit Formaldehyd gegebenenfalls in Gegenwart eines Verdünnungsmittels wie beispielsweise Methanol, Tetrahydrofuran oder Dioxan und gegebenenfalls in Gegenwart eines Reaktionshilfsmittels wie beispielsweise Natriummethylat oder Natronlauge bei Temperaturen zwischen 0 °C und 120 °C umsetzt, wobei in Abhängigkeit von Reaktionsdauer, Reaktionstemperatur und Konzentration an Reaktionshilfsmittel entweder α-Halogenketone der Formel (IX),

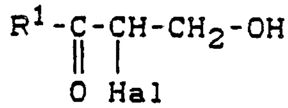
(IX)

in welcher
$R^1$ und Hal die oben angegebene Bedeutung haben,
oder Oxiranylketone der Formel (Vb),

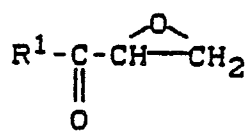
(Vb)

in welcher
$R^1$ die oben angegebene Bedeutung hat,
entstehen, und wenn man diese α-Halogenketone der Formel (IX) oder die Oxiranylketone der Formel (Vb) oder ein Gemisch aus Verbindungen dieser Art in einer 2. Stufe mit Aldehyden oder Ketonen der Formel (VI),

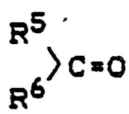
(VI)

in welcher
$R^5$ und $R^6$ die oben angegebene Bedeutung haben,
gegebenenfalls in Gegenwart eines Verdünnungsmittels wie beispielsweise Tetrachlorkohlenstoff und gegebenenfalls in Gegenwart eines Katalysators wie beispielsweise Zinntetrachlorid und gegebenenfalls in Gegenwart eines Säurebindemittels wie beispielsweise Natriummethylat oder Natriumhydroxid bei Temperaturen zwischen - 80 °C und + 80 °C umsetzt (vgl. z.B. Angew. Chem. Int. Ed. Engl. 21, 449 [1982]).

Aldehyde und Ketone der Formel (VI) sind allgemein bekannte Verbindungen der organischen Chemie.

Halogenmethylketone der Formel (VIII) sind ebenfalls allgemein bekannte Verbindungen der organischen Chemie oder erhältlich in Analogie zu allgemein bekannten Verfahren.

Oxiranylketone der Formel (Vb) sind teilweise bekannt (vgl. z.B. Can. J. Chem. 62, 2429-2434 [1984]; Tetrahedron 40, 1381-1390 [1984]; Chem. Lett. 1982, 1601-1604; J. org. Chem. 45, 3407-3413 [1980]; J. org. Chem. 43, 1323-1327 [1978]; Chem. Ber. 108, 2391-2396 [1975]; J. org. Chem. 39, 388-393 [1974]).

α-Halogenketone der Formel (IX) sind ebenfalls teilweise bekannt (vgl. z.B. Tetrahedron Lett. 28, 383-386 [1987]; Synth. Commun. 16, 1517-1522 [1986]; Synthesis 1986, 60-61; Tetrahedron Lett. 23, 4369-4370 [1982]; J. org Chem. 47, 3289-3296 [1982]; Tetrahedron 36, 3101-3105 [1980]; Acta chem. Scand B 34, 41-45 [1980]; J. org. Chem. 33, 2473-2477 [1968]).

Noch nicht bekannt sind α-Halogenketone der Formel (IXa),

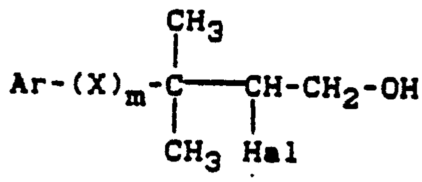

(IXa)

in welcher
Hal für Halogen, insbesondere für Chlor oder Brom steht und
Ar, X und m die oben angegebene Bedeutung haben; sie sind Gegenstand einer parallelen Anmeldung.

Man erhält sie in Analogie zum oben angegebenen allgemeinen Verfahren, wenn man Halogenmethylketone der Formel (VIIIa),

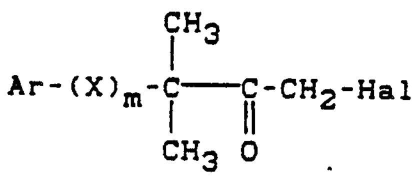

(VIIIa)

in welcher
Ar, X, Hal und m die oben angegebene Bedeutung haben,
mit Formaldehyd gegebenenfalls in Gegenwart eines Verdünnungsmittels wie beispielsweise Methanol, Ethanol, Tetrahydrofuran oder Dioxan und gegebenenfalls in Gegenwart eines Reaktionshilfsmittels wie beispielsweise Natriummethylat bei Temperaturen zwischen 0 °C und 60 °C umsetzt.

Halogenmethylketone der Formel (VIIIa) sind bekannt oder erhältlich in Analogie zu bekannten Verfahren (vgl. z.B. DE-OS 26 32 603; DE-OS 30 21 516; EP 54 865).

Dioxolanylketone der Formel (IIc),

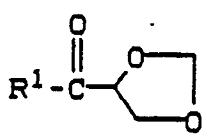

(IIc)

in welcher
$R^1$ die oben angegebene Bedeutung hat,
erhält man alternativ auch, wenn man Halogenmethylketone der Formel (VIII),

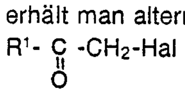 (VIII)

in welcher
$R^1$ die oben angegebene Bedeutung hat und
Hal für Halogen, insbesondere für Chlor oder Brom steht,
mit mindestens zwei Äquivalenten Formaldehyd gegebenenfalls in Gegenwart eines Verdünnungsmittels wie beispielsweise Methanol oder Tetrahydrofuran und gegebenenfalls in Gegenwart eines Säurebindemittels wie beispielsweise Natriummethylat oder Natriumhydroxid bei Temperaturen zwischen 20 °C und 80 °C umsetzt, wobei intermediär auftretende α-Halogenketone der Formel (IX) nicht isoliert werden.

Die zur Durchführung des erfindungsgemäßen Verfahrens weiterhin als Ausgangsstoffe benötigten metallorganischen Heterocyclen sind durch die Formel (III) allgemein definiert. In dieser Formel (III) steht A vorzugsweise für diejenigen Reste, die bereits im Zusammenhang mit der Beschreibung der erfindungsgemäßen Stoffe der Formel (I) als bevorzugt für diesen Substituenten genannt wurden.

M steht vorzugsweise für ein Lithium- oder Natriumkation oder für einen Rest -Mg-Hal, wobei Hal vorzugsweise für Chlor, Brom oder Iod, insbesondere für Brom steht.

Metallorganische Heterocyclen der Formel (III) sind bekannt (vgl. z.B. EP 221 844; Angew. Chem. Int. Ed. Engl. 8, 279 [1969]; EP 131 867).

Als Verdünnungsmittel zur Durchführung des erfindungsgemäßen Verfahrens kommen inerte organische

Lösungsmittel infrage. Hierzu gehören insbesondere aliphatische, alicyclische oder aromatische Kohlenwasserstoffe, wie beispielsweise Benzin, Benzol, Toluol, Xylol, Petrolether, Hexan oder Cyclohexan, Ether, wie Diethylether, Diisopropylether, Dioxan, Tetrahydrofuran oder Ethylenglykoldimethyl- oder -diethylether oder Amide, wie Hexamethylphosphorsäuretriamid.

Die Reaktionstemperaturen können bei der Durchführung des erfindungsgemäßen Verfahrens in einem größeren Bereich variiert werden. Im allgemeinen arbeitet man bei Temperaturen zwischen - 120 °C und + 150 °C, vorzugsweise bei Temperaturen zwischen - 80 °C und + 120 °C.

Zur Durchführung des erfindungsgemäßen Verfahrens setzt man pro Mol an Dioxolanylketon der Formel (II) im allge meinen 1.0 bis 3.0 Mol, vorzugsweise 1.0 bis 1.5 Mol an metallorganischem Heterocylus der Formel (III) ein.

Die Reaktionsdurchführung erfolgt nach allgemein üblichen Methoden.
Dabei kann die Umsetzung erforderlichenfalls in Gegenwart eines geeigneten Inertgases, wie beispielsweise Stickstoff oder Helium durchgeführt werden.
Es ist auch möglich, den als Reaktionspartner eingesetzten metallorganischen Heterocyclus der Formel (III) aus geeigneten Ausgangsverbindungen, wie beispielsweise 5-Brompyrimidin oder 3-Brompyridin und n-Butyllithium oder Isopropylmagnesiumbromid, in einer vorgelagerten Reaktion direkt im Reaktionsgefäß herzustellen und ohne Isolierung im Eintopfverfahren mit dem Dioxolanylketon der Formel (II) weiter umzusetzen.
Die Aufarbeitung und Isolierung der Reaktionsprodukte erfolgt ebenfalls nach üblichen Methoden (vgl. die Herstellungsbeispiele).

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die Metallsalzkomplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren isolieren und gegebenenfalls durch Umkristallisation reinigen.

Darüber hinaus sind die erfindungsgemäßen substituierten Dioxolane der Formel (I) als interessante Zwischenprodukte zur Herstellung von weiteren Wirkstoffen verwendbar.

Sie können beispielsweise an der Hydroxygruppe durch Alkylierung oder Acylierung mit üblichen Alkylhalogeniden oder Alkylsulfaten oder mit Acylhalogeniden oder Carbamoylhalogeniden umgesetzt werden zu entsprechenden Ethern, Estern, Carbonaten oder Carbamaten, welche ebenfalls gute Wirksamkeit gegen Pflanzenschädlinge besitzen.

Die erfindungsgemäßen Wirkstoffe weisen eine starke Wirkung gegen Schädlinge auf und können zur Bekämpfung von unerwünschten Mikroorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytri diomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum; Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;

Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger des Getreidebraunrostes an Weizen (Puccinia recondita) oder gegen den Erreger der Braunspelzigkeit des Weizens (Leptosphaeria nodorum) oder gegen den Erreger der Netzfleckenkrankheit der Gerste (Pyrenophora teres) oder gegen den Erreger der Braunfleckenkrankheit an Gerste und Weizen (Cochliobolus sativus) oder zur Bekämpfung von Reiskrankheiten, wie beispielsweise gegen den Erreger der Reisfleckenkrankheit (Pyricularia oryzae) oder zur Bekämpfung von Krankheiten im Obst- und Gemüseanbau, wie beispielsweise gegen den Erreger des Bohnengrauschimmels (Botrytis cinerea) oder gegen den Erreger des Apfelschorfes (Venturia inaequalis) einsetzen.

Darüberhinaus zeigen die erfindungsgemäßen Wirkstoffe eine gute fungizide in vitro-Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenak tiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabak stengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

## Herstellungsbeispiele

### Beispiel 1

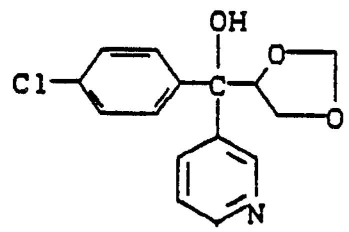

Zu 15,2 g (0,0835 Mol) 3-Pyridylmagnesiumbromid in einem Gemisch aus 50 ml Ether und 35 ml Tetrahydrofuran gibt man bei Raumtemperatur tropfenweise unter Rühren 15 g (0,07 Mol) 4-(4-Chlorbenzoyl)-1,3-dioxolan in 35 ml Tetrahydrofuran, rührt nach beendeter Zugabe 8 Stunden bei Rückflußtemperatur, kühlt auf Raumtemperatur ab, neutralisiert mit verdünnter wässriger Salzsäure, trennt die organische Phase ab, trocknet über Natriumsulfat, engt im Vakuum ein und reinigt den Rückstand durch Säulenchromatographie an Kieselgel (Laufmittel: Essigester/Cyclohexan 3:1).

Man erhält 7,5 g (37 % der Theorie) an 4-Chlorphenyl-3-pyridyl-4-(1,3-dioxolanyl)-carbinol als Öl.
$^1$H-NMR ($CDCl_3$/Tetramethylsilan): $\delta$ = 4,95 (s,1H); 5,07 (s,1H) ppm.

## Herstellung der Ausgangsverbindung

### Beispiel II-1

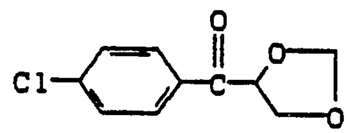

Zu 232 g (0,993 Mol) 4-Chlorphenacylbromid (vgl. z.B. DE-OS 21 41 527 oder Arzneim. Forsch. 22,

2095-2096 [1972]) und 119,1 g (3,97 Mol) Paraformaldehyd in 300 ml Methanol gibt man bei 30 °C tropfenweise unter Rühren 258 g (0,957 Mol) einer 20prozentigen methanolischen Natriummethylatlösung, rührt nach beendeter Zugabe eine Stunde bei Raumtemperatur, engt dann im Vakuum ein, nimmt den Rückstand in Dichlormethan auf, filtriert, engt das Filtrat im Vakuum ein und destilliert den Rückstand im Hochvakuum.

Man erhält 37,5 g (18 % der Theorie) an 4-(4-Chlor-benzoyl)-1,3-dioxolan vom Siedepunkt 153 °C bei 3 mbar und vom Schmelzpunkt 53 °C.

In entsprechender Weise und gemäß den allgemeinen Angaben zur Herstellung erhält man die folgenden substituierten Dioxolane der allgemeinen Formel (I):

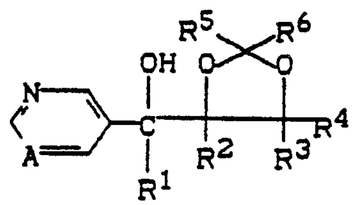

(I)

| Bsp. Nr. | $R^1$ | 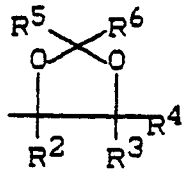 | A | physika-lische Eigen-schaften |
|---|---|---|---|---|
| 2 | $(CH_3)_3C-$ | 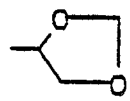 | CH | Fp 127° C |
| 3 | 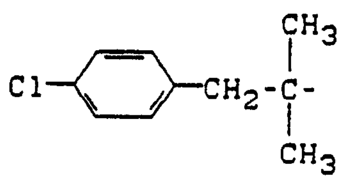 | 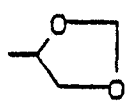 | CH | $^1$H-NMR*): 0,84; 4,97; 4,75 |
| 4 | 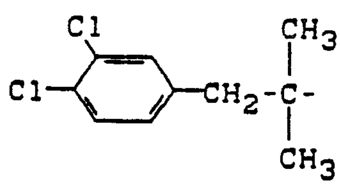 | 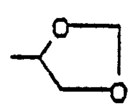 | CH | $^1$H-NMR*): 0,87 |
| 5 | 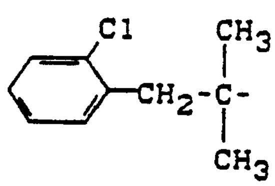 | 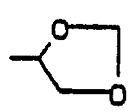 | CH | Fp 85° C |

| Bsp. Nr. | $R^1$ | 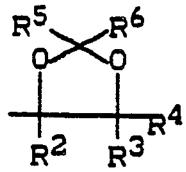 | A | physikalische Eigenschaften |
|---|---|---|---|---|
| 6 | 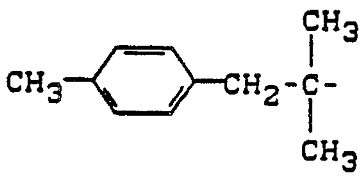 | 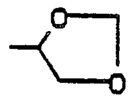 | CH | Fp 58° C |
| 7 | 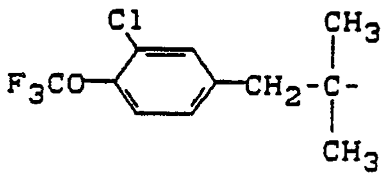 | 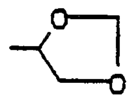 | CH | Fp 124° C |
| 8 | 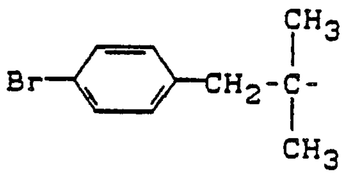 | 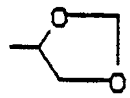 | CH | Fp 68° C |
| 9 | 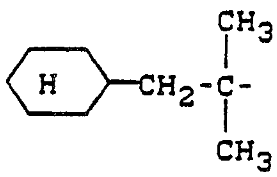 | 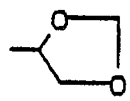 | CH | $^1$H-NMR*): 0,91; 0,92 |
| 10 | 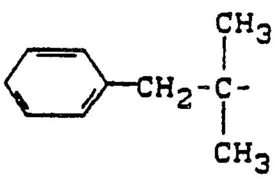 | 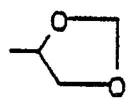 | CH | $n_D^{20}$ 1.5631 |
| 11 | 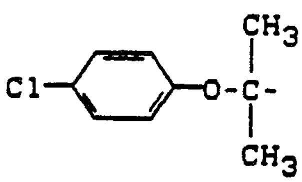 | 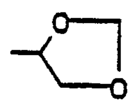 | CH | Fp 109° C |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Anwendungsbeispiele

In den folgenden Anwendungsbeispielen wurden die nachstehend aufgeführten Verbindungen als Vergleichssubstanzen eingesetzt:

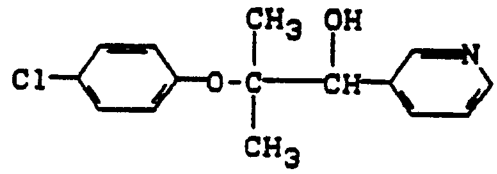

(A)

2-(4-Chlorphenoxy)-2-methyl-1-(3-pyridyl)-1-propanol

(bekannt aus EP 221 844)

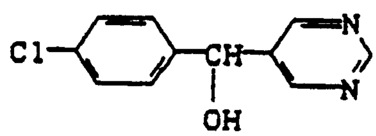

(B)

4-Chlorphenyl-5-pyrimidinyl-methanol

(bekannt aus US 4 417 050)

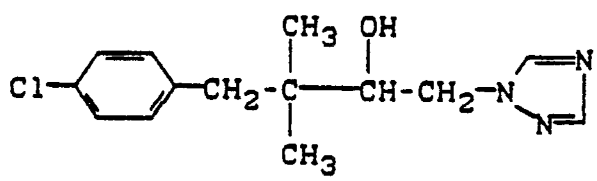

(C)

4-(4-Chlorphenyl)-3,3-dimethyl-1-(1,2,4-triazol-1-yl)-2-butanol

(bekannt aus EP 55 833).

Beispiel A

Puccinia-Test (Weizen) / protektiv

Lösungsmittel: 100 Gewichtsteile Dimethylformamid

Emulgator: 0,025 Gewichtsteile Alkylarylpolyglykolether

Zur Herstellung einer zweckmäßigen Wirkstoffzubereitung vermischt man 1 Gewichsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20 °C und 100 % rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt, um die Entwicklung von Rostpusteln zu begünstigen.

10 Tage nach der Inokulation erfolgt die Auswertung.

Gegenüber der Verbindung A zeigen die Verbindungen der Herstellungsbeispiele 2 und 3 bei einer beispielhaften Konzentration von 0,025 Gew.-% einen um 40 bis 55 % höheren Wirkungsgrad.

## Beispiel B

Leptosphaeria nodorum-Test (Weizen)/protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit gesprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 15 °C und einer relativen Luftfeuchtigkeit von ca. 80 % aufgestellt.

10 Tage nach der Inokulation erfolgt die Auswertung.

Gegenüber der Verbindung A zeigt die Verbindung des Herstellungsbeispiels 3 bei einer beispielhaften Konzentration von 0,0025 Gew.-% einen sehr hohen Wirkungsgrad. Die Verbindung A ist nahezu unwirksam.

## Beispiel C

Pyrenophora teres-Test (Gerste) / protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Abtrocknen des Spritzbelages werden die Pflanzen mit einer Konidiensuspension von Pyrenophora teres besprüht. Die Pflanzen verbleiben 48 Stunden bei 20 °C und 100 % relativer Luftfeuchtigkeit in einer Inkubationskabine.

Die Pflanzen werden in einem Gewächshaus bei eine Temperatur von ca. 20 °C und einer relativen Luftfeuchtigkeit von ca. 80 % aufgestellt.

7 Tage nach der Inokulation erfolgt die Auswertung.

Gegenüber der Verbindung A zeigt die Verbindung des Herstellungsbeispiels 3 bei einer beispielhaften Konzentration von 0,01 Gew.-% einen um 25 % höheren Wirkungsgrad.

Beispiel D

Botrytis-Test (Bohne)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

Gegenüber der Verbindung A zeigt die Verbindung des Herstellungsbeispiels 1 bei einer beispielhaften Konzentration von 100 ppm einen sehr hohen Wirkungsgrad. Die Verbindung A ist nahezu unwirksam.

Beispiel E

Venturia-Test (Apfel) /protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

Gegenüber der Verbindung C zeigen die Verbindungen der Herstellungsbeispiele 1 und 3 bei einer beispielhaften Konzentration von 10 ppm einen sehr hohen Wirkungsgrad. Die Verbindung C ist nahezu unwirksam.

Beispiel F

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Gegenüber der Verbindung B zeigen die Verbindungen der Herstellungsbeispiele 4, 6 und 17 einen sehr hohen Wirkungsgrad. Die Verbindung B ist nahezu unwirksam.

**Ansprüche**

1. Substituierte Dioxolane der allgemeinen Formel (I),

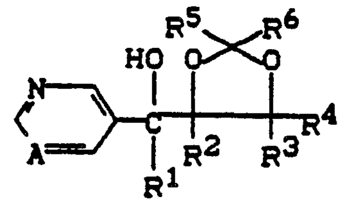

(I)

in welcher
$R^1$ für jeweils unsubstituiertes oder substituiertes Alkyl, Alkenyl, Alkinyl, Cycloalkyl oder Aryl steht,
$R^2$, $R^3$, $R^4$, $R^5$ und $R^6$ unabhängig voneinander jeweils für Wasserstoff, Alkyl oder Cycloalkyl stehen, wobei auch entweder $R^3$ und $R^4$ gemeinsam oder $R^5$ und $R^6$ gemeinsam für einen zweifach verknüpften Alkandiylrest stehen können und
A für Stickstoff oder eine CH-Gruppe steht,
sowie deren Säureadditionssalze und Metallsalzkomplexe.

2. Substituierte Dioxolane gemäß Anspruch 1, wobei in der Formel (I),
$R^1$ für jeweils geradkettiges oder verzweigtes Alkyl mit 1 bis 12 Kohlenstoffatomen, Alkenyl mit 2 bis 12 Kohlenstoffatomen oder Alkinyl mit 2 bis 12 Kohlenstoffatomen, für jeweils geradkettiges oder verzweigtes Alkoxyalkyl, Alkylthioalkyl, Alkylsulfinylalkyl, Alkylsulfonylalkyl, Alkoximinoalkyl, Hydroximinoalkyl oder Cyanalkyl mit jeweils 1 bis 8 Kohlenstoffatomen in den einzelnen Alkylteilen, für jeweils geradkettiges oder verzweigtes Halogenalkyl, Halogenalkoxyalkyl, Halogenalkylthioalkyl, Halogenalkylsulfinylalkyl oder Halogenalkylsulfonylalkyl mit jeweils 1 bis 8 Kohlenstoffatomen in den einzelnen Alkylteilen und jeweils 1 bis 9 gleichen oder verschiedenen Halogenatomen, für jeweils geradkettiges oder verzweigtes Halogenalkenyl oder Halogenalkinyl mit jeweils 2 bis 12 Kohlenstoffatomen und jeweils 1 bis 9 gleichen oder verschiedenen Halogenatomen, für Dioxolanylalkyl, Dithiolanylalkyl, Dioxanylalkyl oder Dithianylalkyl mit jeweils 1 bis 6 Kohlenstoffatomen in geradkettigen oder verzweigten Alkylteil oder für jeweils unsubstituiertes oder im Cycloalkylteil einfach bis mehrfach, gleich oder verschieden substituiertes Cycloalkylalkyl oder Cycloalkyl mit jeweils 3 bis 7 Kohlenstoffatomen im Cycloalkylteil und 1 bis 8 Kohlenstoffatomen im geradkettigen oder verzweigten Alkylteil steht, wobei als Substituenten im Cycloalkylteil jeweils infrage kommen: Halogen oder geradkettiges oder verzweigtes Alkyl mit 1 bis 4 Kohlenstoffatomen; oder für jeweils unsbustituiertes oder im Arylteil einfach bis mehrfach, gleich oder verschieden substituiertes Arylalkyl, Aryloxyalkyl, Arylthioalkyl, Arylsulfinylalkyl, Arylsulfonylalkyl, Aralkyloxyalkyl, Aralkylthioalkyl, Aralkylsulfinylalkyl, Aralkylsulfonylalkyl oder Aryl mit jeweils 6 bis 10 Kohlenstoffatomen im Arylteil und jeweils 1 bis 8 Kohlenstoffatomen in den einzelnen geradkettigen oder verzweigten Alkylteilen steht, wobei als Arylsubstituenten jeweils infrage kommen: Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, unsubstituiertes oder einfach bis mehrfach, gleich oder verschieden durch Halogen subsituiertes, zweifach verknüpftes Dioxyalkylen mit 1 bis 3 Kohlenstoffatomen, Cycloalkyl mit 3 bis 7 Kohlenstoffatomen und unsubstituiertes oder einfach oder mehrfach, gleich oder verschieden durch Halogen und/oder geradkettiges oder verzweigtes Alkyl mit 1 bis 4 Kohlenstoffatomen substituiertes Phenyl oder Phenoxy;
$R^2$, $R^3$, $R^4$, $R^5$ und $R^6$ unabhängig voneinander jeweils für Wasserstoff, für geradkettiges oder verzweigtes Alkyl mit 1 bis 6 Kohlenstoffatomen oder für Cycloalkyl mit 3 bis 7 Kohlenstoffatomen stehen, wobei auch entweder $R^3$ und $R^4$ gemeinsam oder $R^5$ und $R^6$ gemeinsam für einen jeweils zweifach verknüpften Alkandiylrest mit 2 bis 6 Kohlenstoffatomen stehen können und
A für Stickstoff oder eine CH-Gruppe steht,
deren Säureadditionssalze und Metallsalzkomplexe.

3. Substituierte Dioxolane gemäß Anspruch 1, wobei in der Formel (I)
$R^1$ für jeweils geradkettiges oder verzweigtes Alkyl mit 1 bis 8 Kohlenstoffatomen, Alkenyl mit 2 bis 8 Kohlenstoffatomen oder Alkinyl mit 2 bis 8 Kohlenstoffatomen, für jeweils geradkettiges oder verzweigtes Alkoxyalkyl, Alkylthioalkyl, Alkylsulfinylalkyl, Alkylsulfonylalkyl, Alkoximinoalkyl, Hydroximinoalkyl oder Cyanalkyl mit jeweils 1 bis 6 Kohlenstoffatomen in den einzelnen Alkylteilen, für jeweils geradkettiges oder

verzweigtes Halogenalkyl, Halogenalkoxyalkyl, Halogenalkylthioalkyl, Halogenalkylsulfinylalkyl oder Halogenalkylsulfonylalkyl mit jeweils 1 bis 4 Kohlenstoffatomen in den einzelnen Alkylteilen und jeweils 1 bis 5 gleichen oder verschiedenen Halogenatomen, für jeweils geradkettiges oder ver zweigtes Halogenalkenyl oder Halogenalkinyl mit jeweils 3 bis 8 Kohlenstoffatomen und jeweils 1 bis 5 gleichen oder verschiedenen Halogenatomen, für Dioxolanylalkyl, Dithiolanylalkyl, Dioxanylalkyl oder Dithianylalkyl mit jeweils 1 bis 4 Kohlenstoffatomen im geradkettigen oder verzweigten Alkylteil;

oder für jeweils unsubstituiertes oder im Cycloalkylteil ein- bis fünffach, gleich oder verschieden substituiertes Cycloalkylalkyl oder Cycloalkyl mit jeweils 3 bis 6 Kohlenstoffatomen im Cycloalkylteil und 1 bis 6 Kohlenstoffatomen im geradkettigen oder verzweigten Alkylteil steht, wobei als Substituenten im Cycloalkylteil jeweils infrage kommen: Fluor, Chlor, Brom oder geradkettiges oder verzweigtes Alkyl mit 1 bis 4 Kohlenstoffatomen;

oder für jeweils unsubstituiertes oder im Arylteil ein- bis fünffach, gleich oder verschieden substituiertes Arylalkyl, Aryloxyalkyl, Arylthioalkyl, Arylsulfinylalkyl, Arylsulfonylalkyl, Aralkyloxyalkyl, Aralkylthioalkyl, Aralkylsulfinylalkyl, Aralkylsulfonylalkyl oder Aryl mit 1 bis 6 Kohlenstoffatomen in den einzelnen geradkettigen oder verzweigten Alkylteilen steht, wobei Aryl jeweils für Phenyl, α-Naphthyl oder ß-Naphthyl steht, wobei als Phenylsubstituenten jeweils infrage kommen: Fluor, Chlor, Brom, Cyano, Nitro, Methyl, Ethyl, n- oder i-Propyl, n-, i-, s- oder t- Butyl, Methoxy, Ethoxy, n- oder i-Propoxy, Methylthio, Ethylthio, Propan-1,3-diyl, Butan-1,4-diyl, Pentan-1,5-diyl, Dimethylpropan-1,3-diyl, Tetramethylpropan-1,3-diyl, Diemthylbutan-1,4-diyl, Tetramethylbutan-1,4-diyl, Cyclopropyl, Cyclobutyl, Cyclopentyl, Cylohexyl, Trifluormethyl, Dichlorfluormethyl, Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethoxy, Difluormethoxy, Fluormethoxy, Dichlorfluormethoxy, Difluorchlormethoxy, Difluorbrommethoxy, Trichlormethoxy, Trifluorethoxy, Tetrafluorethoxy, Pentafluorethoxy, Trifluorchlorethoxy, Trifluordichlorethoxy, Difluortrichlorethoxy, Pentachlorethoxy, Trifluormethylthio, Difluormethylthio, Fluormethylthio, Difluorchlormethylthio, Dichlorfluormethylthio, Difluorethylthio, Difluorbrommethylthio, Trichlormethylthio, Trifluorethylthio, Tetrafluorethylthio, Pentafluorethylthio, Trifluorchlorethylthio, Trifluordichlorethylthio, Pentachlorethylthio, Methylsulfinyl, Trifluormethylsulfinyl, Dichlorfluormethylsulfinyl, Difluorchlormethylsulfinyl, Fluormethylsulfinyl, Difluormethylsulfinyl, Methylsulfonyl, Trifluormethylsufonyl, Dichlorfluormethylsulfonyl, Difluorchlormethylsulfonyl, Fluormethylsulfonyl, Difluormethylsulfonyl, Methoxycarbonyl, Ethoxycarbonyl, Methoximinomethyl, Ethoximino methyl, Methoximinoethyl, Ethoximinoethyl, Difluordioxymethylen, Tetrafluordioxyethylen, Trifluordioxyethylen, Difluordioxyethylen, Dioxymethylen, Dioxyethylen, jeweils unsbustituiertes oder ein- bis dreifach, gleich oder verschieden durch Methyl, Fluor oder Chlor substituiertes Phenyl oder Phenoxy;

und wobei als Naphthylsubstituenten jeweils infrage kommen: Fluor, Chlor, Brom, Cyano, Methyl, Ethyl, n- oder i-Propyl;

$R^2$, $R^3$, $R^4$, $R^5$ und $R^6$ unabhängig voneinander jeweils für Wasserstoff, Methyl, Ethyl, n-oder i-Propyl, n-, i-, s- oder t-Butyl, für Cyclopentyl oder für Cyclohexyl stehen, wobei auch entweder $R^3$ und $R^4$ gemeinsam oder $R^5$ und $R^6$ gemeinsam für einen jeweils zweifach verknüpften Butan-1,4-diylrest oder Pentan-1,5-diylrest stehen können und

A für Stickstoff oder eine CH-Gruppe steht,

deren Säureadditionssalze und Metallsalzkomplexe.

4. Substituierte Dioxolane gemäß Anspruch 1, wobei in der Formel (I),

$R^1$ für jeweils geradkettiges oder verzweigtes Alkyl mit 1 bis 7 Kohlenstoffatomen, Alkenyl mit 2 bis 7 Kohlenstoffatomen, Alkinyl mit 2 bis 7 Kohlenstoffatomen, für jeweils geradkettiges oder verzweigtes Methoxyalkyl, Dimethoxyalkyl, Trimethoxyalkyl, Methylthioalkyl, Dimethylthioalkyl Trimethylthioalkyl, Methylsulfinylalkyl, Methylsulfonylalkyl, Methoximinoalkyl, Hydroximinoalkyl oder Cyanalkyl mit jeweils 3 bis 6 Kohlenstoffatomen im Alkylteil, für jeweils verzweigtes Halogenalkyl, Halogenmethoxyalkyl, Halogenethoxyalkyl, Halogenmethylthioalkyl, Halogenmethylsulfinylalkyl oder Halogenmethylsulfonylalkyl mit jeweils 1 bis 3 gleichen oder verschiedenen Halogenatomen und jeweils 3 bis 7 Kohlenstoffatomen im Alkylteil, für jeweils geradkettiges oder verzweigtes Halogenalkenyl oder Halogenalkinyl mit jeweils 3 bis 7 Kohlenstoffatomen und jeweils 1 bis 3 gleichen oder verschiedenen Halogenatomen oder für Dioxolanylalkyl, Dithiolanylalkyl, Dioxanylalkyl oder Dithianylalkyl mit jeweils 1 bis 3 Kohlenstoffatomen im geradkettigen oder verzweigten Alkylteil steht;

oder für jeweils unsubstituiertes oder ein- bis fünffach, gleich oder verschieden im Cycloalkylteil durch Fluor, Chlor, Brom oder Methyl substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im geradkettigen oder verzweigten Alkylteil steht;

oder für jeweils unsubstituiertes oder ein- bis dreifach, gleich oder verschieden substituiertes Phenyl, α-Naphthyl oder ß-Naphthyl oder für einen Rest der Formel

$Ar-(X)_m-CH_2-$; 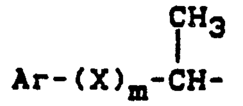 oder 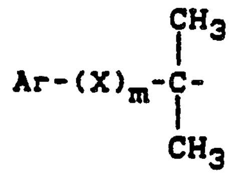 steht,

wobei

Ar jeweils für unsubstituiertes oder ein- bis dreifach, gleich oder verschieden substituiertes Phenyl, α-Naphthyl oder ß-Naphthyl steht,

X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$oder $-S(O)_n-CH_2-CH_2-$ steht,

m jeweils für eine Zahl 0 oder 1 steht und

n jeweils für eine Zahl 0, 1 oder 2 steht,

wobei als Phenylsubstituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Propan-1,3-diyl, Butan-1,4-diyl, Pentan1,5-diyl, Dimethylpropan-1,3-diyl, Tetramethyl propan-1,3-diyl, Dimethylbutan-1,4-diyl, Tetramethylbutan-1,4-diyl, Cyclopropyl, Cyclobutyl, Cyclopentyl, Cylohexyl, Trifluormethyl, Dichlorfluormethyl, Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethoxy, Difluormethoxy, Fluormethoxy, Dichlorfluormethoxy, Difluorchlormethoxy, Difluorbrommethoxy, Trichlormethoxy, Trifluorethoxy, Tetrafluorethoxy, Pentafluorethoxy, Trifluorchlorethoxy, Trifluordichlorethoxy, Difluortrichlorethoxy, Pentachlorethoxy, Trifluormethylthio, Difluormethylthio, Fluormethylthio, Difluorchlormethylthio, Dichlorfluormethylthio, Difluorethylthio, Difluorbrommethylthio, Trichlormethylthio, Trifluorethylthio, Tetrafluorethylthio, Pentafluorethylthio, Trifluorchlorethylthio, Trifluordichlorethylthio, Pentachlorethylthio, Methylsulfinyl, Trifluormethylsulfinyl, Dichlorfluormethylsulfinyl, Difluorchlormethylsulfinyl, Fluormethylsulfinyl, Difluormethylsulfinyl, Methylsulfonyl, Trifluormethylsulfonyl, Dichlorfluormethylsulfonyl, Difluorchlormethylsulfonyl, Fluormethylsulfonyl, Difluormethylsulfonyl, Methoxycarbonyl, Ethoxycarbonyl, Methoximinomethyl, Ethoximinomethyl, Methoximinoethyl, Ethoximinoethyl, Difluordioxymethylen, Tetrafluordioxyethylen, Trifluordioxyethylen, Difluordioxyethylen, Dioxymethylen, Dioxyethylen, unsubstituiertes oder ein- bis dreifach, gleich oder verschieden durch Fluor oder Chlor substituiertes Phenyl oder Phenoxy;

und wobei als Naphthylsubstituenten jeweils infrage kommen: Fluor, Chlor, Brom, Cyano, Methyl, Ethyl, n- oder i-Propyl,

$R^2$, $R^3$, $R^4$, $R^5$ und $R^6$ unabhängig voneinander für Wasserstoff oder Methyl stehen und

A für Stickstoff oder eine CH-Gruppe steht,

deren Säureadditionssalze und Metallsalzkomplexe.

5. Substituierte Dioxolane gemäß Anspruch 1, wobei in der Formel (I),

$R^1$ für jeweils geradkettiges ode verzweigtes Alkyl mit 3 bis 7 Kohlenstoffatomen, Alkenyl mit 3 bis 7 Kohlenstoffatomen oder Alkinyl mit 3 bis 7 Kohlenstoffatomen steht;

oder für jeweils ein- bis dreifach, gleich oder verschieden durch Fluor, Chlor, Methoxy, Ethoxy, Methylthio, Ethylthio, Trifluormethyl, Trifluormethoxy oder Trifluormethylthio substituiertes i-Propyl oder t-Butyl steht;

oder für jeweils einfach durch Cyano, Hydroximino, Methoximino, Ethoximino, Cyclopentyl, Cyclohexyl, Dioxolanyl, Dithiolanyl, Dioxanyl oder Dithianyl substituiertes Methyl, Ethyl, i-Propyl oder t-Butyl steht;

oder für jeweils geradkettiges oder verzweigtes, einfach oder zweifach, gleich oder verschieden durch Fluor, Chlor oder Brom substituiertes Alkenyl oder Alkinyl mit jeweils 3 bis 7 Kohlenstoffatomen steht;

oder für jeweils unsubstituiertes oder ein-bis fünffach, gleich oder verschieden durch Fluor, Chlor, Brom oder Methyl substituiertes Cyclopropyl, Cyclobutyl, Cyclopentyl oder Cyclohexyl steht;

oder für α-Naphthyl oder ß-Naphthyl steht; oder für unsubstituiertes oder ein- bis dreifach, gleich oder verschieden durch Fluor, Chlor, Brom, Methyl, Methoxy, Methylthio, Trifluormethyl, Trifluormethoxy, Trifluormethylthio, Difluormethyl, Difluormethoxy, Difluormethylthio, Phenyl oder Phenoxy substituiertes Phenyl steht;

oder für einen Rest der Formel

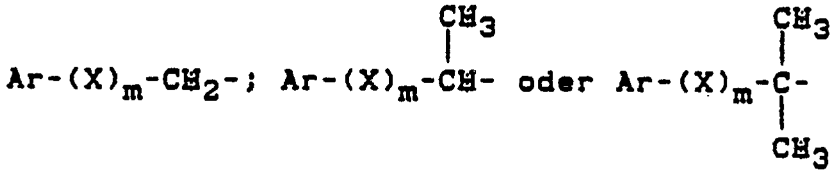 steht,

wobei
Ar jeweils für unsubstituiertes oder ein- bis dreifach, gleich oder verschieden durch Fluor, Chlor, Brom, Methyl, Methoxy, Methylthio, Trifluormethyl, Trifluormethoxy, Trifluormethylthio, Difluormethyl, Difluormethoxy, Difluormethylthio, Phenyl oder Phenoxy substituiertes Phenyl steht oder für jeweils unsubstituiertes oder durch Fluor, Chlor oder Methyl substituiertes α-Naphthyl oder ß-Naphthyl steht,
X für Sauerstoff, Schwefel oder für eine der Gruppen $-CH_2-$, $-O-CH_2-$, $-S-CH_2-$, $-O-CH_2-CH_2-$oder $-S-CH_2-=-CH_2-$ steht und
m für eine Zahl 0 oder 1 steht,
$R^2$, $R^3$, $R^4$, $R^5$ und $R^6$ für Wasserstoff stehen und
A für Stickstoff oder eine CH-Gruppe steht.

6. Verfahren zur Herstellung der Dioxolane der allgemeinen Formel (I),

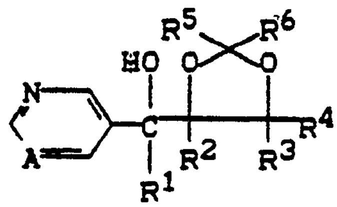 (I)

in welcher
$R^1$ für jeweils unsubstituiertes oder substituiertes Alkyl, Alkenyl, Alkinyl, Cycloalkyl oder Aryl steht,
$R^2$, $R^3$, $R^4$, $R^5$ und $R^6$ unabhängig voneinander jeweils für Wasserstoff, Alkyl oder Cycloalkyl stehen, wobei auch entweder $R^3$ und $R^4$ gemein sam oder $R^5$ und $R^6$ gemeinsam für einen zweifach verknüpften Alkandiylrest stehen können und
A für Stickstoff oder eine CH-Gruppe steht,
sowie deren Säureadditionssalze und Metallsalzkomplexe, dadurch gekennzeichnet, daß man Dioxolanylketone der Formel (II),

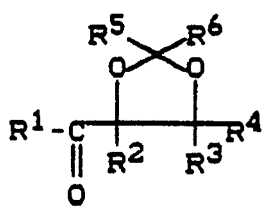 (II)

in welcher
$R^1$, $R^2$, $R^3$, $R^4$, $R^5$ und $R^6$ die oben angegebene Bedeutung haben,
mit metallorganischen Heterocyclen der Formel (III),

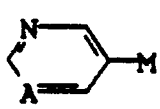 (III)

in welcher
M für ein Alkalimetallkation oder für einen Rest -Mg-Hal steht, wobei
Hal für Halogen steht und

A die oben angegebene Bedeutung hat,
gegebenenfalls in Gegenwart eines Verdünnungsmittels und gegebenenfalls in Gegenwart eines Reaktionshilfsmittels umsetzt und gegebenenfalls anschließend eine Säure oder ein Metallsalz addiert.

7. Verwendung der Dioxolane der Formel (I) gemäß den Ansprüchen 1 bis 6 zur Bekämpfung von Schädlingen.

8. Schädlingsbekämpfungsmittel, gekennzeichnet durch einen Gehalt an mindestens einer Verbindung der Formel (I) gemäß den Ansprüchen 1 bis 6.

9. Verfahren zur Bekämpfung von Schädlingen, dadurch gekennzeichnet, daß man mindestens eine Verbindung der Formel (I) gemäß den Ansprüchen 1 bis 6 auf Schädlinge und/oder ihren Lebensraum einwirken läßt.

10. Verfahren zur Herstellung von Schädlingsbekämpfungsmitteln, dadurch gekennzeichnet, daß man Verbindungen der Formel (I) gemäß den Ansprüchen 1 bis 6 mit Streckmitteln und/oder oberflächenaktiven Mitteln vermischt.

Europäisches Patentamt

# EUROPÄISCHER RECHERCHENBERICHT

Nummer der Anmeldung

EP 89113966.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A2 - 0 209 854 (HOFFMANN) * Formel I; Ansprüche 1,13, 14 * -- | 1,8-10 | C 07 D 405/06<br>A 01 N 43/653<br>A 01 N 43/50 |
| A | EP - B1 - 0 062 238 (HOFFMANN) * Ansprüche 1-4 * -- | 1,8-10 | |
| A | CHEMICAL ABSTRACTS, Band 108, Nr. 13, 28. März 1988, Columbus, Ohio, USA NASU et al. "Preparation of pyridine derivatives as pesticides" Seite 629, Spalte 2, Zusammenfassung-Nr. 112 429b & Jpn. Kokai Tokkyo Koho JP 62,169,766 ---- | 1 | |

| RECHERCHIERTE SACHGEBIETE (Int. Cl.4) |
|---|
| C 07 D 405/00 |

Der vorliegende Recherchenbericht wurde für alle Patentansprüche erstellt

| Recherchenort | Abschlußdatum der Recherche | Prüfer |
|---|---|---|
| WIEN | 16-10-1989 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82